(19)

Europäisches
Patentamt
European
Patent Office
Office européen
des brevets

(11) **EP 2 639 253 A1**

(12) **EUROPEAN PATENT APPLICATION**
published in accordance with Art. 153(4) EPC

(43) Date of publication:
**18.09.2013 Bulletin 2013/38**

(21) Application number: **11840307.0**

(22) Date of filing: **04.11.2011**

(51) Int Cl.:
*C08G 63/06* (2006.01)       *A61K 8/85* (2006.01)
*A61Q 1/00* (2006.01)       *A61Q 5/00* (2006.01)
*A61Q 19/00* (2006.01)       *C07C 69/675* (2006.01)

(86) International application number:
**PCT/JP2011/075400**

(87) International publication number:
**WO 2012/063727 (18.05.2012 Gazette 2012/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.11.2010   JP 2010249415**

(71) Applicant: **The Nisshin OilliO Group, Ltd.
Tokyo 104-8285 (JP)**

(72) Inventors:
• **DENDA, Hirofumi
Yokohama-shi
Kanagawa 235-8558 (JP)**
• **OOKUBO, Minaho
Yokohama-shi
Kanagawa 235-8558 (JP)**

• **NAMIKI, Nobuo
Tokyo 131-8501 (JP)**
• **HANYAMA, Atsushi
Odawara-shi
Kanagawa 250-0002 (JP)**
• **INOUE, Yasuhiko
Odawara-shi
Kanagawa 250-0002 (JP)**

(74) Representative: **Albrecht, Thomas
Kraus & Weisert
Patent- und Rechtsanwälte
Thomas-Wimmer-Ring 15
80539 München (DE)**

(54) **ESTERIFICATION PRODUCT AND COSMETICS**

(57)   Provided is an esterification reaction product having high waterproof-film-forming properties, providing shiny feeling, and being excellent in moisture-retaining properties. The esterification reaction product is prepared by esterifying component A: 12-hydroxystearic acid polymer having a degree of polymerization of 7 or more, component B: fatty acid other than 12-hydroxystearic acid polymers, and component C: pentaerythritol.

EP 2 639 253 A1

## Description

[Technical Field]

The present invention relates to an esterification reaction product, in particular, an esterification reaction product having high waterproof-film-forming properties, providing shiny feeling, and being excellent in moisture-retaining properties.

[Background Art]

Cosmetic products have been used for imparting aesthetic appearance to skin or the like. In addition to the improvement in aesthetic appearance of skin or the like, cosmetic products are also used for preventing evaporation of moisture from the skin to protect the skin from drying and further for treating the skin.
In order to prepare cosmetic products having effects of preventing the skin from drying and of treating the skin, naturally-derived oils having moisturizing properties, such as, lanolin and castor oil, and synthetic ester oils having water-holding properties are widely used as raw materials.
As the naturally-derived oils, lanolin has been widely used. The lanolin is derived from wool grease and shows, for example, high affinity and humectant properties to skin and has excellent adhesion. The lanolin, therefore, has been widely used in basic cosmetics, makeup cosmetics, and many other cosmetics (e.g., see Non-Patent Literature 1).
However, lanolin and castor oil are natural products and therefore have a difficulty in provision of stable quality. In addition, their prices largely fluctuate. Thus, these materials are unstable in the quality and price.
Accordingly, instead of the naturally- derived oils, synthetic ester oils have been used recently. As such synthetic ester oils, ester compounds of 12- hydroxystearic acid and polyols (e.g., see Patent Literatures 1 to 3) and ester compounds of 12- hydroxystearic acid polymers and polyols (e.g., see Patent Literatures 4 to 8) have been developed.
However, the water- holding states of water- in- oil emulsion cosmetic products prepared using the ester compounds disclosed in Patent Literatures 1 to 6 are unstable in some cases to deteriorate the long- term stability and the pigment- dispersing properties of the cosmetic products. Cosmetic products produced using the ester compounds disclosed in Patent Literatures 1 to 6 can well adhere to, for example, skin and also have waterproof- film- forming properties and non- dyeing properties at certain degrees, but further improvements in these characteristics are desired.
Furthermore, the ester compounds described in Patent Literatures 7 and 8 are emulsifiers and therefore do not have a film- forming function, unlike oils. In addition, the usable amounts of these esters in cosmetic products may be restricted depending on types of the cosmetic products.
Furthermore, water- repellent silicone having film- forming properties and thereby being used as a cosmetic raw material hardly provides sheen when it is blended in lipstick or lip gloss, and has a problem in moisturizing properties because of its low moisture- retaining properties. Accordingly, it has been desired to develop a cosmetic oil being excellent in film- forming properties, shiny feeling, and moisture- retaining properties.

[Citation List]

[Patent Literature]

[Patent Literature 1] Japanese Patent Laid-Open No. Sho 54-109917

[Patent Literature 2] Japanese Patent Laid-Open No. Sho 55-57509

[Patent Literature 3] Japanese Patent Laid-Open No. Sho 56-115740

[Patent Literature 4] Japanese Patent Laid-Open No. Sho 56-108739

[Patent Literature 5] Japanese Patent Laid-Open No. Hei 05-331023

[Patent Literature 6] Japanese Patent Laid-Open No. Sho 64-90025

[Patent Literature 7] Japanese Patent Laid-Open No. 2000-290232

[Patent Literature 8] Japanese Patent Laid-Open No. Hei 07-8781

[Non-Patent Literature]

[Non- Patent Literature 1] Keshohin Jiten (Cosmetic Dictionary) , Maruzen, published on December 15, 2003

[Summary of Invention]

[Technical Problem]

Accordingly, it is an object of the present invention to provide an esterification reaction product having high waterproof- film- forming properties, giving shiny feeling, and being excellent in moisture- retaining properties.
It is another object of the present invention to provide an esterification reaction product being excellent in moist feeling and adhesion to, for example, skin or hair, and softening properties and being improved in prevention of makeup deterioration (effects of lasting makeup long and of preventing color migration) , easiness in cleansing, and sense of use (light spreading without stickiness) .
It is another object of the present invention to provide a cosmetic product giving waterproof- film- forming properties, shiny feeling, and moisture- retaining properties, being non- sticky and easy in application, and having excellent moist feeling and makeup- lasting quality.
It is another object of the present invention to provide a method of producing the esterification reaction product.

[Solution to Problem]

In order to achieve the above-mentioned objects, the present inventors have conducted intensive studies and have found that the above-mentioned objects can be achieved by using a specific esterification reaction product. Thus, the present invention has been accomplished.
That is, the present invention relates to an esterification reaction product prepared by esterification of the following components A, B, and C:

Component A: 12-hydroxystearic acid polymer having a degree of polymerization of 7 or more,
Component B: fatty acid other than 12-hydroxystearic acid polymers, and
Component C: pentaerythritol.

The present invention also relates to a cosmetic product containing the esterification reaction product.
The present invention also relates to a method of producing an esterification reaction product. The method includes a step of polymerizing 12-hydroxystearic acid to prepare component A; and a step of esterifying the resulting component A, component B, and component C, wherein
component A is a 12-hydroxystearic acid polymer having a degree of polymerization of 7 or more,
component B is a fatty acid other than 12-hydroxystearic acid polymers, and
component C is pentaerythritol.

[Advantageous Effects of Invention]

The present invention can provide an esterification reaction product having high waterproof-film-forming properties, giving shiny feeling, and being excellent in moisture-retaining properties.
The present invention can also provide an esterification reaction product being excellent in moist feeling, adhesion to, for example, skin or hair, and softening properties and being improved in prevention of makeup deterioration (effects of lasting makeup long and of preventing color migration), easiness in cleansing, and sense of use (light spreading without stickiness).
The present invention can also provide a cosmetic product giving waterproof-film-forming properties, shiny feeling, and moisture-retaining properties, being non-sticky and easy in application, and having excellent moist feeling and makeup-lasting quality.
The present invention can also provide a method of producing the esterification reaction product.

[Description of Embodiments]

The esterification reaction product of the present invention will now be described.
The esterification reaction product of the present invention is prepared by esterifying the following components A to C:

Component A: 12-hydroxystearic acid polymer having a degree of polymerization of 7 or more,

Component B: fatty acid other than 12-hydroxystearic acid polymers, and
Component C: pentaerythritol.

Throughout the specification, the term "degree of polymerization" or "multimer" refers to average degree of polymerization or average number of polymerization.

The method of producing an esterification reaction product of the present invention will now be described. The method of producing an esterification reaction product of the present invention includes a step of esterifying the following components A to C:

Component A: 12-hydroxystearic acid polymer having a degree of polymerization of 7 or more,
Component B: fatty acid other than 12-hydroxystearic acid polymers, and
Component C: pentaerythritol.

The method of producing an esterification reaction product of the present invention is preferably performed by two-stage synthesis. That is, the method of producing an esterification reaction product of the present invention includes a step (first stage) of preparing component A by polymerizing 12-hydroxystearic acid and a step (second stage) of esterifying component A, component B, and component C.

In the esterification reaction product produced by one-stage synthesis, i.e., one-pot synthesis, instead of the two-stage synthesis, the degree of polymerization of the resulting 12-hydroxystearic acid polymer is low. In the one-pot synthesis, 12-hydroxystearic acid, a fatty acid other than 12-hydroxystearic acid polymers, and pentaerythritol are esterified in one-stage. In such a method, the degree of polymerization of 12-hydroxystearic acid is low. Therefore, in the present invention, it is preferred to produce the esterification reaction product by two-stage synthesis.

The degree of polymerization of the 12-hydroxystearic acid polymer used for producing the esterification reaction product of the present invention can be calculated based on the results of acid value measurement.

For example, if the acid value at completion of the esterification stage is 19.8, the molecular weight is 56.11 x 1000/19.8 = 2838, which corresponds to the molecular weight of a decamer of 12-hydroxystearic acid. Accordingly, the resulting polymer has a degree of polymerization of 10. The method of producing an esterification reaction product of the present invention uses a 12-hydroxystearic acid polymer having a degree of polymerization of 7 or more. The use of a 12-hydroxystearic acid polymer having a degree of polymerization of 7 or more can give an esterification reaction product having high waterproof-film-forming properties and enhanced shiny feeling. Though it depends on the purpose of the esterification reaction product of the present invention, the degree of polymerization is 7 or more, preferably 8 or more, and more preferably 9 or more. In addition, though it depends on the purpose of the esterification reaction product of the present invention, the degree of polymerization is preferably 20 or less, more preferably 18 or less, more preferably 16 or less, and most preferably 12 or less. The combination of the upper limit and the lower limit of the degree of polymerization is preferably 7 to 20, more preferably 7 to 18, more preferably 7 to 16, and most preferably 7 to 12. Furthermore, the combination is more preferably 8 to 20, more preferably 8 to 18, more preferably 8 to 16, and most preferably 8 to 12. Furthermore, the combination is preferably 9 to 20, more preferably 9 to 18, more preferably 9 to 16, and most preferably 9 to 12. An esterification reaction product having a degree of polymerization within the above-mentioned range can improve, for example, the sheen, easiness of application, moisture, makeup-lasting quality, and no sticky feeling of a cosmetic product containing the esterification reaction product and can give an appropriate feel and sense of use of the cosmetic product.

The 12-hydroxystearic acid polymer in the esterification reaction product produced by one-pot synthesis is theoretically 3.8- to 7.5-mer. For example, in Production Example 1 described below, a reaction of 15 mol of 12-hydroxystearic acid, 2 mol of behenic acid, and pentaerythritol gives the maximum of theoretical average degree of polymerization shown below. Supporting that 1 mol of pentaerythritol first reacts with 2 mol of behenic acid and then two hydroxy groups of the pentaerythritol react with 15 mol of 12-hydroxystearic acid, the average degree of polymerization of the resulting 12-hydroxystearic acid polymer is as follows:

Average degree of polymerization = 15/2 = 7.5 (multimer).

The minimum of theoretical average degree of polymerization is as follows. Supporting that 1 mol of pentaerythritol (the number of hydroxy groups: 4) first reacts with 15 mol of 12-hydroxystearic acid and then 2 mol of behenic acid reacts, the average degree of polymerization of the resulting 12-hydroxystearic acid polymer is as follows:

Average degree of polymerization = 15/4 = 3.8 (multimer).

That is, the esterification reaction product produced by one-pot synthesis has a degree of polymerization of 3.8 to 7.5.

The 12-hydroxystearic acid polymer used for producing the esterification reaction product of the present invention is a 12-hydroxystearic acid polymer having a degree of polymerization of 7 or more and including one hydroxy group in the molecule and can be prepared, for example, through polymerization of 12-hydroxystearic acid prepared by hydrogenation of ricinoleic acid obtained by hydrolysis of castor seed oil.

The polymerization of 12-hydroxystearic acid is esterification of the hydroxy group or carboxy group in a 12-hydroxystearic acid molecule with the carboxy group or hydroxy group of another 12-hydroxystearic acid molecule, i.e., intermolecular esterification.

The step of preparing 12-hydroxystearic acid polymer having a degree of polymerization of 7 or more by polymerizing 12-hydroxystearic acid will now be described.

The polymerization of 12-hydroxystearic acid can be performed as follows:

12-Hydroxystearic acid is charged in a reaction vessel and is esterified (polymerized) with stirring in the presence or absence of an acid, alkali, or other metal catalyst, preferably in an organic solvent and/or gas that are inert to the reaction, at a temperature of 180 to 220°C for 5 to 30 hours.

The 12-hydroxystearic acid preferably has a purity of 94% by mass or more, more preferably 96% by mass or more, and most preferably 98% by mass or more. Throughout the specification, the purity of the 12-hydroxystearic acid is a value obtained by the method described below.

If the purity of 12-hydroxystearic acid is lower than 94% by mass, the degree of polymerization may be low, which may decrease the ratio of polymers having a degree of polymerization of 7 or more. The 12-hydroxystearic acid may be a commercially available product such as trade name "12-Hydro acid (HP)" manufactured by Kokura Synthetic Industries, Ltd.

An average degree of polymerization of 7 or more can be obtained by controlling the polymerization of 12-hydroxystearic acid by measuring the acid value of the reaction product during the polymerization. That is, the average degree of polymerization of 7 or more can be readily obtained by sampling the reaction product during the polymerization of 12-hydroxystearic acid, measuring the acid value thereof, calculating the average degree of polymerization, and terminating the esterification (polymerization) at the point when an intended average degree of polymerization is obtained. In addition, the 12-hydroxystearic acid polymer can have a degree of polymerization within the above-mentioned range by producing the esterification, reaction product by the method described below.

The step of esterifying component A, prepared as in above, component B, and component C will now be described. The fatty acid (component B) other than 12-hydroxystearic acid polymers used in the present invention may be any fatty acid and may be a saturated fatty acid or an unsaturated fatty acid and may be a linear fatty acid or a branched fatty acid, but is preferably a saturated fatty acid from the viewpoint of, for example, oxidation stability of the compound. Examples of such fatty acids include caproic acid, caprylic acid, 2-ethylhexanoic acid, isononanoic acid, capric acid, lauric acid, myristic acid, palmitic acid, stearic acid, isostearic acid, arachidic acid, behenic acid, lignoceric acid, cerotic acid, montanoic acid, and melissic acid. Among the above-mentioned saturated fatty acids, a linear fatty acid having 16 to 28 carbon atoms is more preferred. Examples of such a fatty acid include palmitic acid, stearic acid, arachidic acid, behenic acid, lignoceric acid, cerotic acid, and montanoic acid. Furthermore, a linear fatty acid having 16 to 22 carbon atoms is most preferred. Examples of such a fatty acid include palmitic acid, stearic acid, arachidic acid, and behenic acid. Furthermore, behenic acid is preferred from the viewpoints of functions such as characteristics, functionality, waterproof-film-forming properties, shiny feeling, and moisture-retaining properties.

In the method of producing an esterification reaction product of the present invention, a 12-hydroxystearic acid polymer having a degree of polymerization of 7 or more (component A) prepared as described above, a fatty acid other than 12-hydroxystearic acid polymers (component B), and pentaerythritol (component C) are esterified.

The pentaerythritol used for producing the esterification reaction product of the present invention may be a commercially available high purity product and preferably has a purity of 90% by mass or more. Examples of usable pentaerythritol include trade name "Pentaerythritol." manufactured by LCY Chemical Corp.

Subsequently, the 12- hydroxystearic acid polymer prepared as in above, the fatty acid other than 12- hydroxystearic acid polymers, and the pentaerythritol are esterified. The charging ratio (molar ratio) of the 12- hydroxystearic acid polymer to the fatty acid other than 12- hydroxystearic acid polymers in the esterification is preferably 35: 65 to 90: 10, more preferably 40: 60 to 75: 25, more preferably 40: 60 to 60: 40, and most preferably 40: 60 to 50: 50. If the molar ratio (%) of the 12- hydroxystearic acid polymer is less than 35%, the waterproof- film- forming properties, shiny feeling, and moisture- retaining properties may not be obtained, whereas a molar ratio of higher than 90% may not give appropriate functionality.

The charging ratio of the molar number of the pentaerythritol to 1 mol of the 12-hydroxystearic acid polymer is preferably 0.1 to 1.0, more preferably 0.2 to 0.9, and most preferably 0.3 to 0.8. If the charging amount of the pentaerythritol is less than the above-mentioned range, the fatty acid may be left not to give an ester compound, whereas a charging ratio of higher than the above-mentioned range may deteriorate the functions such as waterproof-film-forming properties, adhesion to, for example, skin, and moisturizing properties.

As described above, the resulting ester product can have intended appearance, viscosity, and so on by adjusting the charging amounts.

The esterification is specifically performed using the 12-hydroxystearic acid polymer having a degree of polymerization of 7 or more (component A), the fatty acid other than 12-hydroxystearic acid polymers (component B), and the pentaerythritol (component C) as follows. Specifically, the 12-hydroxystearic acid polymer, the fatty acid other than 12-hydroxystearic acid polymers, and the pentaerythritol are placed in a reaction vessel and are esterified in an inert organic solvent and/or gas at 200 to 220°C for 1 to 20 hours, followed by purification treatment to give an esterification reaction product.

The esterification may be performed in the presence of a catalyst, as necessary. Examples of the catalyst include acid catalysts and alkoxides of alkaline earth metals. The acid catalyst or the alkoxide of an alkaline earth metal is preferably used in an amount of about 0.001 to 1.0% by mass to the total mass of the reaction raw materials.

After the reaction, the catalyst and the unreacted raw materials can be removed by known purification treatment such as washing with water, alkali deacidification, or absorption treatment. The resulting reaction product can be further purified by decolorization and deodorization treatment.

Thus, an esterification reaction product can be provided as a colorless to light yellow, odorless, transparent liquid. The resulting esterification reaction product can be used as a component of a cosmetic product described below.

The esterification reaction product of the present invention is produced by the method described above and preferably has a hydroxy value of 10 to 70 mgKOH/g, more preferably 10 to 50 mgKOH/g, and most preferably 10 to 30 mgKOH/g. An esterification reaction product having a hydroxy value of less than 10 mgKOH/g may not have intended waterproof-film-forming properties and shiny feeling. An esterification reaction product having a hydroxy value of higher than 70 mgKOH/g may be difficult to be produced. (It may be also difficult to produce an esterification reaction product having a hydroxy value of less than 10 mgKOH/g.) The esterification reaction product having a hydroxy value within the above-mentioned range can be produced by, for example, adjusting the amounts of the pentaerythritol, the 12-hydroxystearic acid polymer, and the fatty acid other than 12-hydroxystearic acid polymers.

In addition, the esterification reaction product of the present invention preferably has an acid value of 10 mgKOH/g or less, more preferably 8 mgKOH/g or less, more preferably 5 mgKOH/g or less, and most preferably 3 mgKOH/g or less. An acid value of higher than 10 mgKOH/g may cause odor and may not provide intended waterproof-film- forming properties and shiny feeling. Though slight odor may be caused even at an acid value of 3 mgKOH/g, the acid value of the esterification reaction product of the present invention is preferably within the above- mentioned range in consideration of other effects (waterproof- film- forming properties and shiny feeling) . An acid value of less than 0.1 mgKOH/g may increase the manufacturing cost. Accordingly, though it depends on the purpose, the lower limit of the acid value of the esterification reaction product of the present invention is preferably 0.1 mgKOH/g or more and more preferably 0.8 mgKOH/g or more. The combination of the upper limit and the lower limit of the acid value is preferably 0.1 to 10 mgKOH/g, preferably 0.8 to 10 mgKOH/g, more preferably 0.1 to 3 mgKOH/g, and most preferably 0.8 to 3 mgKOH/g, from the viewpoint of the cost.

The acid value of the esterification reaction product can be controlled within the above- mentioned range by sampling the reaction product during the esterification, measuring the acid value thereof, and terminating the esterification at the point when an intended acid value is obtained.

Furthermore, since the esterification reaction product of the present invention is produced by esterification of a 12-hydroxystearic acid polymer, a fatty acid other than 12- hydroxystearic acid polymers, and pentaerythritol as described above, the composition of constituent fatty acids (fatty acid residues) does not vary between before and after the esterification. Accordingly, the component ratio of fatty acids of the resulting esterification reaction product is the same as that of the charged amounts of the 12- hydroxystearic acid polymer and the fatty acid other than 12- hydroxystearic acid polymers for esterification, and the molar ratio (%) of the 12- hydroxystearic acid polymer having a degree of polymerization of 7 or more in the fatty acid residues of the esterification reaction product is preferably 35 to 90%, more preferably 40 to 75%, more preferably 40 to 60%, and more preferably 40 to 50%. In addition, the molar ratio (%) of the fatty acid other than 12- hydroxystearic acid polymers is preferably 10 to 65%,  more preferably 25 to 60%, more preferably 40 to 60%, and most preferably 50 to 60%. The molar ratios (%) of the 12- hydroxystearic acid polymer having a degree of polymerization of 7 or more and the fatty acid other than 12- hydroxystearic acid polymers within the above- mentioned ranges can provide functions such as waterproof- film- forming properties, adhesion to, for example, skin, and moisturizing properties.

A cosmetic product of the present invention will now be described.

The cosmetic product of the present invention contains the esterification reaction product of the present invention.

The cosmetic product of the present invention may be used in any purpose and in any formulation, and examples of the cosmetic product include lip cosmetic products, foundations, emollient creams, milky lotions, bases, hair creams, shampoos, hair rinses, hair conditioners, hand creams, beauty essences, eye-area cosmetic products, nail cosmetic products, and sunscreen cosmetic products.

These cosmetic products may be produced by any method, and known methods can be employed.

Since the cosmetic product of the present invention contains the esterification reaction product, among these cosmetic products, the makeup cosmetics, such as lip cosmetic products, bases, foundations, eye-area cosmetic products, and nail cosmetic products, are particularly excellent in moisture-retaining properties, moist feeling, and adhesion to skin and can provide an effect of preventing makeup deterioration (effects of lasting makeup long and of preventing color migration), easiness in cleansing, and, in particular, shiny feeling and sense of use (light spreading without Stickiness). The bases and foundations (liquid foundations, powder foundations, stick foundations, cream foundations, and concealers) can smoothen the rough skin surface by the waterproof-film-forming properties to allow the foundations to be smoothly applied onto the skin and also exhibit effects of softening and moisturizing the skin.

In addition, the present invention can exhibit enhanced durability to sebum, perspiration, and rubbing and an excellent long-lasting makeup effect and can be therefore applied to, for example, sunscreening agents. In the lip cosmetic products (lipstick, lip gloss, and lip cream), eye-area cosmetic products (eyeliner, mascara, eye shadow, and eyebrow pencil), and nail cosmetic products (nail enamel, base coat, and top coat), the excellent adhesion exhibits an effect of maintaining the uniform cosmetic film. In general, a pigment blended in a lip cosmetic product may cause troubles (e.g., roughening, drying, allergy, and darkening) in the skin or mucous membrane by dyeing. However, the cosmetic product containing the esterification reaction product of the present invention can prevent the skin or lip from being dyed by the pigment and can have satisfactory makeup-lasting quality and causes less bleeding. Since the esterification reaction product of the present invention exhibits these effects, in particular, the esterification reaction product can be preferably used in lip cosmetic products. Furthermore, moisturizing and treating effects on lips can be realized by adding, for example, a moisturizing agent, an inorganic salt, an organic salt, a water-soluble agent, or an extract from an animal or plant to the lip cosmetic products. Similarly, in solid powder-type and oil-based foundations, it is possible to impart a moisturizing effect to the foundation by blending, for example, a moisturizing agent, an inorganic salt, an organic salt, a water-soluble agent, or an extract form an animal or plant with the foundation. Furthermore, the esterification reaction product of the present invention can be added to the oil phase of an oil-in-water emulsion cosmetic product.

Sunscreen cosmetic products include water-in-oil (W/O type) cream sunscreen cosmetic products, water-in-oil (W/O type) emulsion sunscreen cosmetic products, water-in-oil (W/O type) multi-layered emulsion sunscreen cosmetic products, oil-in-water (O/W type) cream sunscreen cosmetic products, and oil-in-water (O/W type) emulsion sunscreen cosmetic products, and these sunscreen cosmetic products can particularly have excellent long-acting effect, adhesion, and film-forming feeling to skin by containing the esterification reaction product of the present invention. The sunscreen cosmetic products containing the ester compound described above are excellent in long-acting effect, adhesion, and film-forming feeling to skin and also have high ultraviolet protective properties and excellent makeup-lasting quality.

Though the content of the esterification reaction product in a cosmetic product of the present invention varies depending on the formulation of the cosmetic product, in order to further improve the waterproof-film-forming properties, the moisture-retaining properties, and the shiny feeling of the cosmetic product, the content of the esterification reaction product is preferably 0.1 to 80% by mass, more preferably 1 to 80% by mass, more preferably 1 to 60% by mass, more preferably 1 to 40% by mass, and most preferably 5 to 40% by mass.

The cosmetic product of the present invention optionally contains various components that are usually used in cosmetic products within a range that does not impair the effects of the present invention. Though it varies depending on the purpose and the formulation of the cosmetic product, examples of the component include oily components, aqueous components, polymer emulsions, anionic surfactants, cationic surfactants, amphoteric surfactants, lipophilic nonionic surfactants, hydrophilic nonionic surfactants, natural surfactants, moisturizing agents, thickeners, preservatives, powder components, pigments, pH adjusters, antioxidants, ultraviolet absorbers, perfumes, dyes, sequestering agents, and purified water.

Examples of the oily component include hydrocarbons such as liquid paraffin, heavy liquid isoparaffin, solid paraffin, $\alpha$- olefin oligomer, squalane, vaseline, polyisobutylene, polybutene, montan wax, ceresin wax, microcrystalline wax, polyethylene wax, and Fischer- Tropsch wax; oils and fats such as olive oil, castor oil, jojoba oil, mink oil, and macadamia nut oil; waxes such as bees wax, sperm whale, candelilla wax, carnauba wax, and Japan wax; esters such as cetyl 2- ethylhexanoate, cetyl isooctanoate, isopropyl myristate, isopropyl palmitate, octyldodecyl myristate, polyglyceryl diisostearate, polyglyceryl triisostearate, diglyceryl triisostearate, polyglyceryl tetraisostearate, diglyceryl tetraisostearate, trioctanoin, diisostearyl malate, neopentyl glycol dioctanoate, propylene glycol didecanoate, cholesterol fatty acid ester, isopropyl myristate, glyceryl monostearate, glycerin fatty acid ester eicosadioate condensate, dextrin palmitate, dextrin myristate, and dextrin fatty acid ester; fatty acids such as stearic acid, lauric acid, myristic acid, behenic acid, isostearic acid, and oleic acid; higher alcohols such as stearyl alcohol, cetyl alcohol, lauryl alcohol, oleyl alcohol, isostearyl alcohol, behenyl alcohol, stearyl alcohol, octyldodecanol, and isohexadecyl alcohol; silicones such as low-polymerized dimethylpolysiloxane, highly- polymerized dimethylpolysiloxane, methylphenylpolysiloxane, decamethylcyclopentasiloxane, octamethylcyclotetrasiloxane, polyether- modified polysiloxane, polyoxyalkylene/ alkylmethylpolysiloxane/ methylpolysiloxane copolymer, and alkoxy- modified polysiloxane; fluorinated oils such as perfluorodecane, perfluorooctane, and perfluoropolyether; amino acid ester oils such as N- acylglutamic acid, e.g., stearoylglutamic acid, and di (cholesteryl or phytosteryl/ behenyl/ octyldodecyl) N- lauroyl- L- glutamate; and lanolin derivatives such as lanolin, liquid lanolin, lanolin acetate, liquid lanolin acetate, lanolin fatty acid isopropyl ester, and lanolin alcohol. These oily components may be used alone or in a combination of two or more thereof.

Examples of the aqueous component include lower alcohols such as ethyl alcohol and butyl alcohol; glycols such as propylene glycol, 1,3-butylene glycol, dipropylene glycol, and polyethylene glycol; glycerols such as glycerin, diglycerin, and polyglycerin; and plant extracts such as aloe vera, witch hazel, hammamelis, cucumber, tomato, apple, lemon, lavender, and rose. These aqueous components may be used alone or in a combination of two or more thereof.

Examples of the polymer emulsion include alkyl acrylate copolymer emulsions, alkyl methacrylate polymer emulsions, alkyl acrylate copolymer emulsions, alkyl methacrylate copolymer emulsions, acrylic acid/alkyl acrylate copolymer emulsions, methacrylic acid/alkyl methacrylate copolymer emulsions, alkyl acrylate/styrene copolymer emulsions, alkyl methacrylate/styrene copolymer emulsions, vinyl acetate polymer emulsions, polyvinyl acetate emulsions, vinyl acetate-containing copolymer emulsions, vinylpyrrolidone/styrene copolymer emulsions, and silicone-containing copolymer emulsions. These polymer emulsions may be used alone or in a combination of two or more thereof.

Examples of the anionic surfactant include soap base materials; fatty acid soap such as sodium laurate and sodium palmitate; higher alkyl sulfate esters such as sodium lauryl sulfate and potassium lauryl sulfate; alkyl ether sulfate esters such as triethanolamine polyoxyethylene (POE)- lauryl sulfate and sodium POE- lauryl sulfate; N- acylsarcosinates such as sodium lauroylsarcosinate; higher fatty acid amide sulfonates such as sodium N- myristoyl- N- methyl taurine, sodium coconut fatty acid methyltauride, and sodium lauryl methyltauride; phosphate esters such as sodium POE-oleylether phosphate and POE- stearylether phosphate; sulfosuccinates such as sodium di- 2- ethylhexyl sulfosuccinate, sodium monolauroyl monoethanolamide polyoxyethylene sulfosuccinate, and sodium lauryl polypropylene glycol sulfosuccinate; alkylbenzenesulfonates such as sodium linear dodecylbenzenesulfonate, triethanolamine linear dodecylbenzenesulfonate, and linear dodecylbenzenesulfonic acid; N- acylglutamates such as monosodium N- lauroyl glutamate, disodium N- stearoyl glutamate, and monosodium N- myristoyl- L- glutamate; higher fatty acid ester sulfate esters such as sodium hydrogenated coconut fatty acid glyceryl sulfate; sulfated oils such as Turkey red oil; POE alkyl ether carboxylates; POE alkylallyl ether carboxylate; α- olefin sulfonates; higher fatty acid ester sulfonates; secondary alcohol sulfate esters; higher fatty acid alkylolamide sulfate esters; sodium lauroyl monoethanolamide succinate; di- triethanolamine N- palmitoylaspartate; and casein sodium. These anionic surfactants may be used alone or in a combination of two or more thereof.

Examples of the cationic surfactants include alkyltrimethylammonium salts such as stearyltrimethylammonium chloride and lauryltrimethylammonium chloride; dialkyldimethylammonium salts such as distearyldimethylammonium chloride; alkylpyridinium salts such as poly (N, N'- dimethyl- 3, 5- methylenepiperidinium chloride) and cetylpyridinium chloride; alkyl quaternary ammonium salts; alkyldimethylbenzylammonium salts; alkylisoquinolinium salts; dialkylmorpholinium salts; POE- alkylamines; alkylamine salts; polyamine fatty acid derivatives; amyl alcohol fatty acid derivatives; benzalkonium chloride; and benzethonium chloride. These cationic surfactants may be used alone or in a combination of two or more thereof.

Examples of the amphoteric surfactants include imidazoline amphoteric surfactants such as sodium 2- undecyl-N, N, N- (hydroxyethylcarboxymethyl)- 2- imidazoline and 2- cocoyl- 2- imidazoliniumhydroxide- 1- carboxyethyloxy- 2-sodium salt; and betaine surfactants such as 2- heptadecyl- N- carboxymethyl- N- hydroxyethylimidazolinium betaine, lauryldimethylaminoacetic acid betaine, alkylbetaine, amidobetaine, and sulfobetaine. These amphoteric surfactants may be used alone or in a combination of two or more thereof.

Examples of the lipophilic nonionic surfactant include sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerolsorbitan penta- 2- ethylhexylate, and diglycerolsorbitan tetra- 2- ethylhexylate; sucrose fatty acid esters; glyceryl fatty acids such as glyceryl monocotton seed fatty acid, glyceryl monoerucate, glyceryl sesquioleate, glyceryl monostearate, glyceryl $\alpha$, $\alpha$'- oleate pyroglutamate, and glyceryl monostearate; polyglyceryl fatty acid esters such as diglyceryl monoisostearate and diglyceryl diisostearate; propylene glycol fatty acid esters such as propylene glycol monostearate; hydrogenated castor oil derivatives; and glycerin alkylethers. These lipophilic nonionic surfactants may be used alone or in a combination of two or more thereof.

Examples of the hydrophilic nonionic surfactants include POE-sorbitan fatty acid esters such as POE-sorbitan monooleate, POE-sorbitan monostearate, and POE-sorbitan tetraoleate; POE-sorbitol fatty acid esters such as POE-sorbitol monolaurate, POE-sorbitol monooleate, POE-sorbitol pentaoleate, and POE-sorbitol monostearate; POE-glycerin fatty acid esters such as POE-glycerin monostearate, POE-glycerin monoisostearate, and POE-glycerin triisostea-rarate; POE-fatty acid esters such as POE-monooleate, POE-distearate, POE-monodioleate, and ethylene glycol distearate; POE-alkyl ethers such as POE-lauryl ether, POE-oleyl ether, POE-stearyl ether, POE-behenyl ether, POE-2-octyldodecyl ether, and POE-cholestanol ether; pluronic type surfactants such as Pluronic; POE/POP alkyl ethers such as POE/POP-cetyl ether, POE/POP-2-decyltetradecyl ether, POE/POP-monobutyl ether, POE/POP-hydrogenated lanolin, and POE/POP-glycerin ether; tetra POE/tetra POP-ethylenediamine polymers such as Tetronic; POE-castor oil hydrogenated castor oil derivatives such as POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, and POE-hydrogenated castor oil maleate; POE-bees wax/lanolin derivatives such as POE-sorbitol bees wax; alkanol amides such as coconut oil fatty acid diethanolamide, lauric acid monoethanolamide, and fatty acid isopropanolamide; POE-propylene glycol fatty acid esters; POE-alkylamines; POE-fatty acid amides; sucrose fatty acid esters; POE-nonylphenyl formaldehyde polymers; alkylethoxydimethylamine oxides; and trioleyl phosphate. These hydrophilic nonionic surfactants may be used alone or in a combination of two or more thereof.

Examples of the natural surfactants include lecithins such as soybean phospholipid, hydrogenated soybean phospholipid, yolk phospholipid, and hydrogenated yolk phospholipid; and soybean saponin. These natural surfactants may be used alone or in a combination of two or more thereof.

Examples of the moisturizing agents include polyethylene glycol, propylene glycol, glycerin, 1, 3- butylene glycol, xylitol, sorbitol, maltitol, chondroitin sulfate, hyaluronic acid, mucoitinsulfuric acid, caronic acid, atherocollagen, cholesteryl- 12- hydroxystearate, sodium lactate, urea, bile acid salts, dl- pyrrolidone carboxylic acid salts, short- chain soluble collagen, diglycerol (EO) PO adducts, Rosa roxburghii extracts, Achillea millefolium extracts, and melilot extracts. These moisturizing agents may be used alone or in a combination of two or more thereof.

Examples of the thickeners include gum Arabic, carrageenan, karaya gum, tragacanth gum, carob gum, quince seeds (marmelo), casein, dextrin, gelatin, sodium pectate, sodium alginate, methylcellulose, ethylcellulose, CMC, hydroxyethylcellulose, hydroxypropylcellulose, PVA, PVM, PVP, sodium polyacrylate, carboxyvinyl polymer, locust bean gum, guar gum, tamarind gum, dialkyldimethylammonium cellulose sulfate, xanthan gum, magnesium aluminum silicate, bentonite, hectorite, quaternary ammonium caution-modified bentonite, quaternary ammonium cation-modified hectorite, and decaglycerin fatty acid ester eicosadioate condensate. These thickeners may be used alone or in a combination of two or more thereof.

Examples of the preservatives include methylparaben, ethylparaben, and butylparaben. These preservatives may be used alone or in a combination of two or more thereof.

Examples of the powder components include inorganic powders such as talc, kaolin, mica, sericite, white mica, gold mica, synthetic mica, red mica, black mica, rithia mica, vermicurite, magnesium carbonate, calcium carbonate, aluminum silicate, barium silicate, calcium silicate, magnesium silicate, strontium silicate, tungstate metal salts, magnesium, silica, zeolite, barium sulfate, calcined calcium sulfate (calcined gypsum), calcium phosphate, fluorine apatite, hydroxyapatite, ceramic powder, metallic soap (zinc myristate, calcium palmitate, and aluminum stearate), and boron nitride; and organic powders such as polyamide resin powder (nylon powder), polyethylene powder, methyl polymethacrylate powder, polystyrene powder, styrene-acrylic acid copolymer resin powder, benzoguanamine resin powder, poly (ethylene tetrafluoride) powder, and cellulose powder. These powder components may be used alone or in a combination of two or more thereof.

Examples of the pigments include inorganic white pigments, such as titanium dioxide and zinc oxide, (including fine particles of titanium dioxide or zinc oxide which are used as ultraviolet-scattering agents and surface-coated inorganic white pigments given by coating the surfaces of the titanium dioxide or zinc oxide particles with fatty acid soap (e.g., aluminum stearate or zinc palmitate), fatty acid (e.g., stearic acid, myristic acid, or palmitic acid), or fatty acid ester (e.g., dextrin palmitate)); inorganic red pigments such as iron oxide (bengara) and iron titanate; inorganic brown pigments such as $\gamma$-iron oxide; inorganic yellow pigments such as yellow iron oxide and yellow ocher; inorganic black pigments such as black iron oxide, carbon black, and lower order titanium oxide; inorganic violet pigments such as mango violet and cobalt violet; inorganic green pigments such as chromium oxide, chromium hydroxide, and cobalt titanate; inorganic blue pigments such as ultramarine blue and iron blue; pearl pigments such as titanium oxide-coated mica, titanium oxide-coated bismuth oxychloride, titanium oxide-coated talc, colored titanium oxide-coated mica, bismuth oxychloride, and argentine foil; metal powder pigments such as aluminum powder and copper powder; organic pigments such as Red Nos. 201, 202, 204, 205, 220, 226, 228, and 405, Orange Nos. 203 and 204, Yellow Nos. 205 and 401, and Blue No. 404; and organic pigments such as zirconium, barium, and aluminum lakes, e.g., Red Nos. 3, 104, 106, 227, 230, 401, and 505, Orange No. 205, Yellow Nos. 4, 5, 202, and 203, Green No. 3, and Blue No. 1. These pigments may be used alone or in a combination of two or more thereof.

Examples of the pH-adjusters include edetic acid, disodium edetate, citric acid, sodium citrate, sodium hydroxide, potassium hydroxide, and triethanolamine. These pH-adjusters may be used alone or in a combination of two or more thereof.

Examples of the antioxidants include vitamin C, its derivatives, and salts thereof; tocopherols, their derivatives, and salts thereof; dibutylhydroxy toluene; butylhydroxy anisole; and gallic acid esters. These antioxidants may be used alone or in a combination of two or more thereof.

Examples of the ultraviolet absorbers include benzoate ultraviolet absorbers such as p- aminobenzoic acid (hereinafter referred to as PABA) , PABA monoglycerin ester, N, N- dipropoxy PABA ethyl ester, N, N- diethoxy PABA ethyl ester, N, N- dimethyl PABA ethyl ester, N, N- dimethyl PABA butyl ester, and N, N- dimethyl PABA octyl ester; anthranilic acid ultraviolet absorbers such as homomethyl- N- acetyl anthranilate; salicylic acid ultraviolet absorbers such as amyl salicylate, menthyl salicylate, homomenthyl salicylate, octyl salicylate, phenyl salicylate, benzyl salicylate, and p- isopropanolphenyl salicylate; cinnamic acid ultraviolet absorbers such as octyl cinnamate, ethyl 4- isopropylcinnamate, methyl 2, 5- diisopropylcinnamate, ethyl 2, 4- diisopropylcinnamate, methyl 2, 4- diisopropylcinnamate, propyl p- methoxycinnamate, isopropyl p- methoxycinnamate, isoamyl p- methoxycinnamate, octyl p- methoxycinnamate (2- ethylhexyl p- methoxycinnamate) , 2- ethoxyethyl p- methoxycinnamate, cyclohexyl p- methoxycinnamate, ethyl $\alpha$- cyano- $\beta$- phenylcinnamate, 2- ethylhexyl $\alpha$- cyano- $\beta$- phenylcinnamate, and glyceryl mono- 2- ethylhexanoyl diparamethoxycinnamate; benzophenone ultraviolet absorbers such as 2, 4- dihydroxybenzophenone, 2, 2'- dihydroxy- 4- methoxybenzophenone, 2, 2'- dihydroxy- 4, 4'- dimethoxybenzophenone, 2, 2', 4, 4'- tetrahydroxybenzophenone, 2- hydroxy- 4- methoxybenzophenone, 2- hydroxy- 4- methoxy- 4'- methylbenzophenone, 2- hydroxy- 4- methoxybenzophenone- 5- sulfonate, 4- phenylbenzophenone, 2- ethylhexyl- 4'- phenylbenzophenone- 2- carboxylate, 2- hydroxy- 4- n- octoxybenzophenone, and 4- hydroxy- 3- carboxybenzophenone; 3- (4'- methylbenzylidene)- d, l- camphor; 3- benzylidened, l- camphor; urocanic acid; ethyl urocanate ester; 2- phenyl- 5- methylbenzoxazole; 2, 2'- hydroxy- 5- methylphenylbenzotriazole; 2- (2'- hydroxy- 5'- t- octylphenyl) benzotriazole; 2- (2'- hydroxy- 5'- methylphenyl) benzotriazole; dibenzalazine; dianisoylmethane; 4- methoxy- 4'- t- butyldibenzoylmethane; 5- (3, 3- dimethyl- 2- norbornylidene)- 3- pentan- 2- one; 2, 4, 6- trianilino- p- (carbo- 2'- ethylhexyl- l'- oxy) l, 3, 5- triazine; and 4-- tert- butyl- 4'- methoxydibenzoylmethane. These ultraviolet absorbers may be used alone or in a combination of two or more thereof.

Examples of the dyes include chlorophyll and $\beta$-carotene. These dyes may be used alone or in a combination of two or more thereof.

Examples of the perfumes include plant perfumes such as rose oil, jasmine oil, and lavender oil; and synthetic perfumes such as limonene, citral, linalool, and eugenol. These perfumes may be used alone or in a combination of two or more thereof.

Examples of the sequestering agents include disodium edetate, edetic acid salts, and hydroxyethane diphosphonate. These sequestering agents may be used alone or in a combination of two or more thereof.

[Examples]

The present invention will now be further described in detail by Examples. However, it is apparent that the present invention is not limited to the Examples. In the following Examples, part(s) and % denote part(s) by mass and % by mass, respectively, unless otherwise specified.

Further, the acid value and hydroxy value of esterification reaction products were measured in accordance with the Japanese Standards of Quasi-drug Ingredients 2006.

The purity of 12-hydroxystearic acid in the following Reference Examples was measured as follows:

Preparation of sample

A sample was placed in a test tube, and a trimethylsilylating (TMS) agent (TMS-HT: HMDS and TMCS in anhydrous pyridine) was added to the tube. The mixture was heated at 80°C with well stirring for 10 minutes, followed by leaving to stand for 20 minutes. One milliliter of hexan was added to the reaction solution, and the mixture was sufficiently mixed. After centrifugation, the hexane layer (upper layer) was collected and was subjected to gas chromatography (GC) under the following analytical conditions:

Column: DB-5HT, 0.32 mm x 15 m, 0.10 $\mu$m,
Oven temperature condition: 50°C $\rightarrow$ 340°C, 10°C/min, and
Vaporizing chamber/FID detector temperature:

340°C/350°C.

Reference Example 1

A 1-L four-neck flask equipped with a stirrer, a thermometer, a nitrogen gas-introducing tube, and a water remover was charged with 540 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19 mgKOH/g. The average degree of polymerization calculated from the acid value was 10.

Reference Example 2

A 12-hydroxystearic acid polymer was prepared as in Reference Example 1 except that 12-hydroxystearic acid having a purity of 95% by mass (trade name: 12-Hydro acid HP, manufactured by Kokura Synthetic Industries, Ltd., having a purity of 99% by mass was diluted with stearic acid to a purity of 95% by mass) was used. The acid value of the resulting polymer was measured to be 19 mgKOH/g. The average degree of polymerization calculated from the acid value was 10.

Reference Example 3

A 12-hydroxystearic acid polymer was prepared as in Reference Example 1 except that 12-hydroxystearic acid having a purity of 90% by mass (trade name: Hydroxystearic acid, manufactured by Kokura Synthetic Industries, Ltd., purity: 90% by mass) was used. The acid value of the resulting polymer was measured to be 31 mgKOH/g. The average degree of polymerization calculated from the acid value was 6.

Reference Example 4

The molecular weight distribution of each 12-hydroxystearic acid polymer prepared in Reference Examples 1 to 3 was measured, and the ratio of molecules having a degree of polymerization of 7 or more was determined. The molecular weight distribution was calculated from the ratios of peak areas obtained by liquid chromatography. The analytical conditions were as follows:

Column: connected four styrene-divinylbenzene GPC columns each having an inner diameter of 7.8 mm, a length of 300 mm, and a thickness of 5 $\mu$m,
Mobile phase: tetrahydrofuran,
Column temperature: 40°C,
Flow rate of mobile phase: 0.5 mL/min, and
Detection: differential refractive index (RI).

The results of measurement of molecular weight distribution are shown in Table 1.
[Table 1]

| | Ratio of molecules having a degree of polymerization of 7 or more (%) |
|---|---|
| Reference Example 1 | 77 |
| Reference Example 2 | 76 |
| Reference Example 3 | 22 |

As obvious from the results shown in Table 1, the ratios of molecules having a degree of polymerization of 7 or more were high, 76% and 77%, when 12-hydroxystearic acid having a purity of 95% or 99% was used, whereas the ratio of molecules having a degree of polymerization of 7 or more was 2% when 12-hydroxystearic acid having a purity of 90% by mass was used. The results demonstrate that it is preferable to use 12-hydroxystearic acid having a high purity as a raw material for producing the esterification reaction product of the present invention.

Production Example 1: Production of pentaerythrityl tetra-poly-12--hydroxystearate/behenate (OHV = 21)

A 1-L four-neck flask equipped with a stirrer, a thermometer, a nitrogen gas-introducing tube, and a water remover was charged with 540 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.4 mgKOH/g, and the average degree of polymerization calculated from the acid value was 10.2. Accordingly, the resulting product was a decamer of 12-hydroxystearic acid.
Subsequently, 514 g of the thus- prepared 12- hydroxystearic acid polymer (acid value: 19.4 mgKOH/g, decamer) , 82 g of behenic acid (trade name: PRIFRAC2989, manufactured by Uniqema GmbH) , and 16 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1.4 mgKOH/g or less. After completion of the reaction, the catalyst was removed by filtration. Subsequently, the product was decolorized with activated clay and then deodorized by a common method to give 246 g of an esterification reaction product (pentaerythrityl tetra- poly- 12- hydroxystearate/ behenate (OHV = 21) ) as a paste at room temperature. The resulting esterification reaction product had an acid value of 1.6 mgKOH/g and a hydroxy value of 21 mgKOH/g. In addition, the molar ratio (%) of the 12- hydroxystearic acid polymer having a degree of polymerization of 7 or more was 43%, and the molar ratio (%) of behenic acid was 57%, in the fatty acid residues of the resulting esterification reaction product.

Production Example 2: Production of pentaerythrityl tetra-poly-12-hydroxystearate/behenate (OHV = 60, polyhydroxystearic acid decamer, addition molar ratio (polyhydroxystearic acid: behenic acid = 1.5:2)

A 1-L four-neck flask equipped with a stirrer, a thermometer, a nitrogen gas-introducing tube, and a water remover was charged with 540 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.3 mgKOH/g. The average degree of polymerization calculated from the acid value was 10.2. Accordingly, the resulting product was a decamer of 12-hydroxystearic acid.

Subsequently, 514 g of the thus- prepared 12- hydroxystearic acid polymer (acid value: 19.3 mgKOH/g, decamer) , 82 g of behenic acid (trade name: PRIFRAC2989, manufactured by Uniqema GmbH) , and 27 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 0.7 mgKOH/g or less. After completion of the reaction, the catalyst was removed by filtration. Subsequently, the product was decolorized with activated clay and then deodorized to give 256 g of an esterification reaction product (pentaerythrityl tetra- poly- 12- hydroxystearate/ behenate (OHV = 60) ) as a paste at room temperature. The resulting esterification, reaction product had an acid value of 0.8 mgKOH/g and a hydroxy value of 60 mgKOH/g. In addition, the molar ratio (%) of the 12- hydroxystearic acid polymer having a degree of polymerization of 7 or more was 43%, and the molar ratio (%) of behenic acid was 57%, in the fatty acid residues of the resulting esterification reaction product.

Production Example 3: Production of pentaerythrityl tetra-poly-12-hydroxystearate/behenate

A 1-L four-neck flask equipped with a stirrer, a thermometer, a nitrogen gas-introducing tube, and a water remover was charged with 540 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.0 mgKOH/g. The average degree of polymerization calculated from the acid value was 10.4. Accordingly, the resulting product was a decamer of 12-hydroxystearic acid.

Subsequently, 513 g of the thus- prepared 12- hydroxystearic acid polymer (acid value: 19.4 mgKOH/g, decamer) , 25 g of behenic acid (trade name: PRIFRAC2989, manufactured by Uniqema GmbH) , and 10 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1 mgKOH/g or Less. After completion of the reaction, the catalyst was removed by filtration. Subsequently, the product was decolorized with activated clay and then deodorized by a common method to give 256 g of esterification reaction product (pentaerythrityl tetra- poly- 12- hydroxystearate/ behenate) as a paste at room temperature. The resulting esterification reaction product had an acid value of 1.4 mgKOH/g and a hydroxy value of 13 mgKOH/g. In addition, the molar ratio (%) of the 12- hydroxystearic acid polymer having a degree of polymerization of 7 or more was 71%, and the molar ratio (%) of behenic acid was 29%, in the fatty acid residues of the resulting esterification reaction product.

Production Example 4: Production of pentaerythrityl tetra-poly-12-hydroxystearate/behenate

A 1-L four-neck flask equipped with a stirrer, a thermometer, a nitrogen gas-introducing tube, and a water remover was charged with 430 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.0 mgKOH/g. The average degree of polymerization calculated from the acid value was 10.4. Accordingly, the resulting product was a decamer of 12-hydroxystearic acid.

Subsequently, 396 g of the thus- prepared 12- hydroxystearic acid polymer (acid value: 19.0 mgKOH/g, decamer) , 122 g of behenic acid (trade name: PRIFRAC2989, manufactured by Uniqema GmbH) , and 20 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1 mgKOH/g or less. After completion of the reaction, the catalyst was removed by filtration. Subsequently, the product was decolorized with activated clay and then deodorized by a common method to give 389 g of an esterification reaction product (pentaerythrityl tetra- poly- 12- hydroxystearate/ behenate) as a paste at room temperature. The resulting esterification reaction product had an acid value of 1.1 mgKOH/g and a hydroxy value of 16 mgKOH/g. In addition, the molar ratio (%) of the 12-hydroxystearic acid polymer having a degree of polymerization of 7 or more was 29%, and the molar ratio (%) of behenic acid was 71%, in the fatty acid residues of the resulting esterification reaction product.

Production Example 5: Production of pentaerythrityl tetra-poly-12-hydroxystearate/behenate (OHV = 31)

A 1-L four-neck flask equipped with a stirrer, a thermometer, a nitrogen gas-introducing tube, and a water remover was charged with 505 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.6 mgKOH/g. The average degree of polymerization calculated from the acid value was 10.2. Accordingly, the resulting product was a decamer of 12-hydroxystearic acid.

Subsequently, 481 g of the thus- prepared 12- hydroxystearic acid polymer (acid value: 19.6 mgKOH/g, decamer) , 75 g of behenic acid (trade name: PRIFRAC2989, manufactured by Uniqema GmbH) , and 20 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1.5 mgKOH/g or less. After completion of the reaction, the catalyst was removed by filtration. Subsequently, the product was decolorized with activated clay and then deodorized by a common method to give 473 g of an esterification reaction product (pentaerythrityl tetra- poly- 12- hydroxystearate/ behenate (OHV = 31) ) as a paste at room temperature. The resulting esterification reaction product had an acid value of 1.5 mgKOH/g and a hydroxy value of 31 mgKOH/g. In addition, the molar ratio (%) of the 12- hydroxystearic acid polymer having a degree of polymerization of 7 or more was 43%, and the molar ratio (%) of behenic acid was 57%, in the fatty acid residues of the resulting esterification reaction product.

Producction Example 6: Production of pentaerythrityl tetra-poly-12-hydroxystearate/behenate (OHV = 49)

A 1-L four-neck flask equipped with a stirrer, a thermometer, a nitrogen gas-introducing tube, and a water remover was charged with 501 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.6 mgKOH/g. The average degree of polymerization calculated from the acid value was 10.2. Accordingly, the resulting product was a decamer of 12-hydroxystearic acid.

Subsequently, 473 g of the thus- prepared 12- hydroxystearic acid polymer (acid value: 19.6 mgKOH/g, decamer) , 75 g of behenic acid (trade name: PRIFRAC2989, manufactured by Uniqema GmbH) , and 24 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1.2 mgKOH/g or less. After completion of the reaction, the catalyst was removed by filtration. Subsequently, the product was decolorized with activated clay and then deodorized by a common method to give 403 g of an esterification reaction product (pentaerythrityl tetra- poly- 12- hydroxystearate/ behenate (OHV = 49) ) as a paste at room temperature. The resulting esterification reaction product had an acid value of 1.2 mgKOH/g and a hydroxy value of 49 mgKOH/g. In addition, the molar ratio (%) of the 12- hydroxystearic acid polymer having a degree of polymerization of 7 or more was 43%, and the molar ratio (%) of behenic acid was 57%, in the fatty acid residues of the resulting esterification reaction product.

Production Example 7: Production of pentaerythrityl tetra-poly-12-hydroxystearate/behenate (AV = 7.6)

A 1-L four-neck flask equipped with a stirrer, a thermometer, a nitrogen gas-introducing tube, and a water remover was charged with 508 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.8 mgKOH/g. The average degree of polymerization calculated from the acid value was 10.0. Accordingly, the resulting product was a decamer of 12-hydroxystearic acid.

Subsequently, 490 g of the thus- prepared 12- hydroxystearic acid polymer (acid value: 19.8 mgKOH/g, decamer) , 77 g of behenic acid (trade name: PRIFRAC2989, manufactured by Uniqema GmbH) , and 15 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 7.6 mgKOH/g or less. After completion of the reaction, the catalyst was removed by filtration. Subsequently, the product was decolorized with activated clay and then deodorized by a common method to give 485 g of an esterification reaction product (pentaerythrityl tetra- poly- 12- hydroxystearate/ behenate (AV = 7.6) ) as a paste at room temperature. The resulting esterification reaction product had an acid value of 7.6 mgKOH/g and a hydroxy value of 13 mgKOH/g. In addition, the molar ratio (%) of the 12- hydroxystearic acid polymer having a degree of polymerization of 7 or more was 43%, and the molar ratio (%) of behenic acid was 57%, in the fatty acid residues of the resulting esterification reaction product.

Production Example 8: Production of pentaerythrityl tetra-poly-12-hydroxystearate/behenate (number of polymerization: 16-mer)

A 1-L four-neck flask equipped with a stirrer, a thermometer, a nitrogen gas-introducing tube, and a water remover was charged with 539 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 12.4 mgKOH/g. The average degree of polymerization calculated from the acid value was 16.0. Accordingly, the resulting product was a 16-mer of 12-hydroxystearic acid.

Subsequently, 519 g of the thus- prepared 12- hydroxystearic acid polymer (acid value: 12.4 mgKOH/g, 16-mer) , 51 g of behenic acid (trade name: PRIFRAC2989, manufactured by Uniqema GmbH) , and 10 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 2.8 mgKOH/g or less. After completion of the reaction, the catalyst was removed by filtration. Subsequently, the product was decolorized with activated clay and then deodorized by a common method to give 456 g of an esterification reaction product (pentaerythrityl tetra- poly- 12- hydroxystearate/ behenate (number of polymerization: 16- mer) as a paste at room temperature. The resulting esterification reaction product had an acid value of 2.8 mgKOH/g and a hydroxy value of 15 mgKOH/g. In addition, the molar ratio (%) of the 12- hydroxystearic acid polymer having a degree of polymerization of 7 or more was 43%, and the molar ratio (%) of behenic acid was 57%, in the fatty acid residues of the resulting esterification reaction product.

Production Example 9: Production of pentaerythrityl tetra-poly-12-hydroxystearate/palmitate

A 1-L four-neck flask equipped with a stirrer, a thermometer, a nitrogen gas-introducing tube, and a water remover was charged with 524 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.7 mgKOH/g. The average degree of polymerization calculated from the acid value was 10.1. Accordingly, the resulting product was a decamer of 12-hydroxystearic acid.

Subsequently, 505 g of the thus- prepared 12- hydroxystearic acid polymer (acid value: 19.7 mgKOH/g, decamer) , 60 g of palmitic acid (trade name: PALMAC98- 16, manufactured by Acidchem Co., Ltd.) , and 16 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1.5 mgKOH/g or less.

After completion of the reaction, the catalyst was removed by filtration. Subsequently, the product was decolorized with activated clay and then deodorized by a common method to give 468 g of an esterification reaction product (pentaerythrityl tetra- poly- 12- hydroxystearate/ palmitate as a paste at room temperature. The resulting esterification reaction product had an acid value of 1.5 mgKOH/g and a hydroxy value of 18 mgKOH/g. In addition, the molar ratio (%) of the 12-hydroxystearic acid polymer having a degree of polymerization of 7 or more was 43%, and the molar ratio (%) of palmitic acid was 57%, in the fatty acid residues of the resulting esterification reaction product.

Production Comparative Example 1: Production of pentaerythrityl tetra-poly-12-hydroxystearate

A 1-L four-neck flask equipped with a stirrer, a thermometer, a nitrogen gas-introducing tube, and a water remover was charged with 540 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 15 hours to give a 12-hydroxystearic acid polymer. The acid value of the resulting polymer was measured to be 19.2 mgKOH/g. The average degree of polymerization calculated from the acid value was 10.3. Accordingly, the resulting product was a decamer of 12-hydroxystearic acid.
Subsequently, 510 g of the thus- prepared 12- hydroxystearic acid polymer (acid value: 19.2 mgKOH/g, decamer) and 70 g of pentaerythritol (trade name: Pentaerythritol, manufactured by LCY Chemical Corp.) were placed in a 1-L four- neck flask equipped with a stirrer, a thermometer, a nitrogen gas- introducing tube, and a water remover, followed by a reaction under nitrogen gas flow with removing generated water at 210°C until the acid value of the product reached 1.8 mgKOH/g or less.
After completion of the reaction, the catalyst was removed by filtration. Subsequently, the product was decolorized with activated clay and then deodorized by a common method to give 382 g of an esterification reaction product (pentaerythrityl tetra- poly- 12- hydroxystearate) as a paste at room temperature. The resulting esterification reaction product had an acid value of 2.0 mgKOH/g and a hydroxy value of 19 mgKOH/g. In this production example, since the raw materials did not include behenic acid, the molar ratio (%) of 12- hydroxystearic acid polymer having a degree of polymerization of 7 or more was 100%, and the molar ratio (%) of behenic acid was 0%, in the fatty acid residues of the resulting esterification reaction product.

Production Comparative Example 2: Production of pentaerythrityl tetra-poly-12-hydroxystearate/behenate

A 1-L four-neck flask equipped with a stirrer, a thermometer, a nitrogen gas-introducing tube, and a water remover was charged with 450 g of 12-hydroxystearic acid (trade name: 12-Hydro acid HP, manufactured by Kokura Synthetic Industries, Ltd., purity: 99% by mass), and tin chloride as a catalyst and xylol as a reflux solvent were added thereto in amounts of 0.1% and 5%, respectively, of the total charged amount. A reaction was performed under nitrogen gas flow with removing generated water at 200°C for 6 hours to give a 12-hydroxystearic acid polymer. The acid value of the resulting polymer was measured to be 32.4 mgKOH/g. The average degree of polymerization calculated from the acid value was 6.1. Accordingly, the resulting product was a hexamer of 12-hydroxystearic acid.
Subsequently, 412 g of the thus- prepared 12- hydroxystearic acid polymer (acid value: 32.4 mgKOH/g, hexamer) , 113 g of behenic acid (trade name: PRIFRAC2989, manufactured by Uniqema GmbH) , and 23 g of pentaerythritol (trade name: Pentaerythritol, manufactured by LCY Chemical Corp.) were placed in a 1- L four- neck flask equipped with a stirrer, a thermometer, a nitrogen gas- introducing tube, and a water remover, followed by a reaction under nitrogen gas flow with removing generated water at 210°C until the acid value of the product reached 1 mgKOH/g or less.
After completion of the reaction, the catalyst was removed by filtration. Subsequently, the product was decolorized with activated clay and then deodorized by a common method to give 389 g of an esterification reaction product (pentaerythrityl tetra- poly- 12- hydroxystearate/ behenate) as a paste at room temperature. The resulting esterification reaction product had an acid value of 1.1 mgKOH/g and a hydroxy value of 16 mgKOH/g. In addition, the molar ratio (%) of the 12-hydroxystearic acid polymer having a degree of polymerization of 7 or more was 43%, and the molar ratio (%) of behenic acid was 57%, in the fatty acid residues of the resulting esterification reaction product.
The esterification reaction products prepared in Production Examples 1 to 9 and Production Comparative Examples 1 and 2 were evaluated by the following methods. In evaluation (1) "oil resistance test", octyl oxystearate (trade name: Salacos EH, manufactured by The Nisshin OilliO Group, Ltd.), dipentaerythrite fatty acid ester (12-hydroxystearic acid : isostearic acid : hydrogen = 3:1:2, trade name: Cosmol 168EV, manufactured by The Nisshin OilliO Group, Ltd.), dipentaerythrityl tri-polyhydroxystearate (trade name: Salacos WO-6, manufactured by The Nisshin OilliO Group, Ltd.), and trimethylsiloxysilicate (water-repellent silicone, trade name: X-21-5250, manufactured by Shin-Etsu Chemical Co., Ltd.) were also tested.
In evaluation (2) "bleeding test", dipentaerythrityl tri-polyhydroxystearate (trade name: Salacos WO-6, manufactured by The Nisshin OilliO Group, Ltd.), octyl oxystearate (trade name: Salacos EH, manufactured by The Nisshin OilliO Group, Ltd.), dipentaerythrite fatty acid ester (12-hydroxystearic acid : isostearic acid : hydrogen = 3:1:2, trade name: Cosmol 168EV, manufactured by The Nisshin OilliO Group, Ltd.), and trimethylsiloxysilicate (water-repellent silicone, trade name: X-21-5250, manufactured by Shin-Etsu Chemical Co., Ltd.) were also tested.

In evaluation (3) "moisture permeability test", octyl oxystearate (trade name: Salacos EH, manufactured by The Nisshin OilliO Group, Ltd.), dipentaerythrite fatty acid ester (12-hydroxystearic acid : isostearic acid : hydrogen = 3:1:2, trade name: Cosmol 168EV, manufactured by The Nisshin OilliO Group, Ltd.), dipentaerythrityl tri-polyhydroxystearate (trade name: Salacos WO-6, manufactured by The Nisshin OilliO Group, Ltd.), trimethylsiloxysilicate (water-repellent silicone, trade name: X-21-5250, manufactured by Shin-Etsu Chemical Co., Ltd.), diisostearyl malate (trade name: Cosmol 222, manufactured by The Nisshin OilliO Group, Ltd.), and diglyceryl triisostearate (trade name: Cosmol 43V, manufactured by The Nisshin OilliO Group, Ltd.) were also tested.

In evaluation (4) "conductance measurement test", octyl oxystearate (trade name: Salacos EH, manufactured by The Nisshin OilliO Group, Ltd.), dipentaerythrite fatty acid ester (12-hydroxystearic acid : isostearic acid : hydrogen = 3:1:2, trade name: Cosmol 168EV, manufactured by The Nisshin OilliO Group, Ltd.), dipentaerythrityl tripolyhydroxystearate (trade name: Salacos WO-6, manufactured by The Nisshin OilliO Group, Ltd.), trimethylsiloxysilicate (water-repellent silicone, trade name: X-21-5250, manufactured by Shin-Etsu Chemical Co., Ltd.), diglyceryl triisostearate (trade name: Cosmol 43V, manufactured by The Nisshin OilliO Group, Ltd.), and diisostearyl malate (trade name: Cosmol 222, manufactured by The Nisshin OilliO Group, Ltd.) were also tested.

Evaluation (1) "oil resistance test"

The esterification reaction products prepared in Production Examples 1 to 9 and Production Comparative Examples 1 and 2 and the above-mentioned test materials were each mixed with a pigment base [Red No. 202 (manufactured Kishi Kasei Co., Ltd.): 5%, Red No. 201 (manufactured Kishi Kasei Co., Ltd.): 10%, titanium oxide (trade name: JA-C, manufactured by Tayca Corporation): 30%, synthetic mica (trade name: Micromica MK-200K, manufactured by Co-op Chemical Co., Ltd.): 5%, diisostearyl malate (trade name: Cosmol 222, manufactured by The Nisshin OilliO Group, Ltd.): 50%] at a weight ratio of 2:8 to prepare samples. Five milligrams of each sample was applied onto a piece of artificial leather (Supplale (registered trademark), Idemitsu Technofine Co., Ltd.) having a length of 4 cm and a width of 3 cm, and the leather was attached to the inside of a beaker. Macadamia nut oil warmed to 40°C was poured into the beaker such that the sample-applied portion was immersed in the oil, and was stirred in a bath at 40°C for 10 minutes. After 10 minutes, the conditions of the sample remaining on the leather were visually evaluated. The evaluation results are shown in Table 2. In this test, a sample that was appropriately removed is determined to be good. Accordingly, in the results shown in Table 2, "Good" means that the sample was appropriately removed, and "Poor" means that the sample was not removed or was drastically removed. The evaluation results are shown in Table 2.
Incidentally, if a sample is drastically removed, a cosmetic product containing the sample is supposed to be poor in makeup-lasting quality, and if a sample is not removed, a cosmetic product containing the sample is supposed not to be easily removed. Accordingly, a sample that is appropriately removed is evaluated to be good. Evaluation (2) "Bleeding test"

A lipstick was produced with the composition shown below according to the following method.
Diglyceryl triisostearate (trade name: Cosmol 43V, manufactured by The Nisshin OilliO Group, Ltd.): 21%, diisostearyl malate (trade name: Cosmol 222, manufactured by The Nisshin OilliO Group, Ltd.): 20%, glyceryl try-2-ethylhexanoate (trade name: T.I.O, manufactured by The Nisshin OilliO Group, Ltd.): 20%, dipentaerythrite fatty acid ester (12-hydroxystearic acid : isostearic acid : rosin = 4:1.5:0.5, trade name: Cosmol 168ARV, manufactured by The Nisshin OilliO Group, Ltd.): 12%, trehalose isostearate esters (trade name: Nomcort TQ-5, manufactured by The Nisshin OilliO Group, Ltd.): 5%, candelilla wax: 3%, ceresin: 6%, carnauba wax: 2%, microcrystalline wax: 1%, and the pigment base (the same as that in evaluation (1)): 10% were weighed and heated in a water bath at 90°C, followed by stirring with a disper mixer for 5 minutes. After deaeration, the mixture was molded, cooled, and demolded to produce a lipstick.
The resulting lipstick was applied to six zones of the same artificial skin as that used in the oil resistance test to draw a 2-cm square in each zone. Furthermore, the esterification reaction products prepared in Production Examples 1 and 2 and the above-mentioned test materials were each applied on the respective squares in an amount of 0.3 g, followed by being left to stand in a thermostatic chamber at 40°C for 1 hour. Then, the conditions of the lipstick were observed and were evaluated in accordance with the following evaluation criteria for whether bleeding was caused or not. The evaluation results are shown in Table 2.

Good: no bleeding was observed,
Moderate: slight bleeding was observed, and
Poor: considerable bleeding was observed.

Evaluation (3) "moisture permeability test"

Sealing wax (bees wax : paraffin = 60: 40) was melted. Separately, the esterification reaction product prepared in Production Example 1 and the above- mentioned test materials were each poured in the respective petri dishes and were dried in a thermostatic chamber at 100°C. The weight (a) of filter paper No. 5C was measured, and the filter paper was immersed in the sample in the petri dish. The filter paper was pulled up, and excess sample was wiped off. Subsequently, the weight (b) of the filter paper impregnated with the sample was measured, and the sample application amount expressed by the following equation was adjusted to be 6 to 8 mg/cm$^2$.

Sample application amount (mg/cm$^2$) = (b- a) / 38.47 (area of the filter paper) .

Subsequently, about 20 mL (about 15 g) of glycerin was put in a moisture permeation cup. The filter paper applied with the sample was set to the cup with a fixing frame, and the circumference of the moisture permeation cup was sealed with the sealing wax. (Separately, a blank cup was similarly prepared.) After solidification of the sealing wax, the weight (c) of the moisture permeation cup was measured, taking care to avoid adhering of the glycerin to the filter paper. Subsequently, the moisture permeation cup was left to stand in a desiccator saturated with water vapor for 3 hours (25°C) . The moisture permeation cup was then taken out from the desiccator, and the weight (d) of the cup was measured. The moisture permeability of the sample was calculated by the following equation:

$$\text{Permeated water amount } (mg/cm^2) = (d-c)/28.26 \text{ (the}$$

$$\text{permeation area of the moisture permeation cup),}$$

$$\text{Moisture permeability } (\%) = [ \text{(moisture permeability of}$$

$$\text{sample)/(moisture permeability of blank)}] \times 100.$$

A lower moisture permeability means higher moisture- retaining properties of the cosmetic product containing the sample. The evaluation results are shown in Table 2. Evaluation (4) "conductance measurement test"

The upper arm inside of a female normal subject was wiped with ethanol, and the conductance thereof was measured with a high- frequency conductivity sensor (SKICON- 200, manufactured by I.B.S Co., Ltd.) in a room at a temperature of 20 to 25°C and a relative humidity of 50 to 55%. Separately, the esterification reaction products prepared in Production Examples 1 to 9 and Production Comparative Examples 1 and 2 and the above- mentioned test materials were each applied in an appropriate amount to the upper arm inside wiped with ethanol in a 1.5- cm square. After leaving to stand for 90 to 120 minutes, the conductance of the upper arm inside was measured. The average of values obtained by measuring 10 subjects was used as the measured value. The value obtained by subtracting the measured value before the application of the sample from the measured value after the application was defined as the resistance value. The measurement results are shown in Table 3. The unit of the values in Table 3 is $\mu$S.

[Table 2-1]

| Sample | | Evaluation (1) Oil resistance test | Evaluation (2) Bleeding test | Evaluation (3) Moisture permeability test |
|---|---|---|---|---|
| Example 1 | Esterification reaction product (Production Example 1) | Good | Good | 2.16 |
| Example 2 | Esterification reaction product (Production Example 2) | Good | Good | 3.61 |
| Example 3 | Esterification reaction product (Production Example 3) | Good | Good | 1.47 |
| Example 4 | Esterification reaction product (Production Example 4) | Poor (sample was not removed) | Good | 2.42 |

(continued)

| Sample | | Evaluation (1) Oil resistance test | Evaluation (2) Bleeding test | Evaluation (3) Moisture permeability test |
|---|---|---|---|---|
| Example 5 | Esterification reaction product (Production Example 5) | Good | Good | 2.48 |
| Example 6 | Esterification reaction product (Production Example 6) | Good | Good | 3.21 |
| Example 7 | Esterification reaction product (Production Example 7) | Good | Good | 2.72 |
| Example 8 | Esterification reaction product (Production Example 8) | Good | Good | 1.10 |
| Example 9 | Esterification reaction product (Production Example 9) | Good | Good | 1.97 |

[Table 2-2]

| Sample | | Evaluation (1) Oil resistance test | Evaluation (2) Bleeding test | Evaluation (3) Moisture permeability test |
|---|---|---|---|---|
| Comparative Example 1 | Esterification reaction product (Production Comparative Example 1) | Poor (sample was not removed) | Good | 1.22 |
| Comparative Example 2 | Esterification reaction product (Production Comparative Example 2) | Poor (sample was drastically removed) | Moderate | 3.61 |
| Comparative Example 3 | Octyl oxystearate | Poor (sample was not removed) | Poor | 5.59 |
| Comparative Example 4 | Dipentaerythrite fatty acid ester (12-hydroxystearic acid : isostearic acid : hydrogen = 3:1:2) | Poor (sample was not removed) | Good | 3.96 |
| Comparative Example 5 | Dipentaerythrityl tri-polyhydroxystearate | Poor (sample was drastically removed) | Moderate | 2.89 |
| Comparative Example 6 | Trimethylsiloxysilicate (water-repellent silicone) | Poor (sample was not removed) | Good | 11.73 |
| Comparative Example 7 | Diisostearyl malate | - | - | 4.48 |
| Comparative Example 8 | Diglyceryl triisostearate | - | - | 7.87 |

As obvious from the results shown in Table 2, the esterification reaction products prepared in Production Examples 1 to 3 and 5 to 9 showed satisfactory results in the oil resistance test. In addition, it was revealed that the esterification reaction products prepared in Production Examples 1 to 9 have excellent properties for preventing bleeding. Furthermore, the esterification reaction products prepared in Production Examples 1 to 9 showed satisfactory results in the moisture permeability test.

[Table 3-1]

| Sample | | Before application | After application | Resistance value |
|--------|--|--------------------|------------------|------------------|
| Example 1 | Esterification reaction product (Production Example 1) | 103 | 343 | 240 |
| Example 2 | Esterification reaction product (Production Example 2) | 114 | 365 | 251 |
| Example 3 | Esterification reaction product (Production Example 3) | 111 | 330 | 219 |
| Example 4 | Esterification reaction product (Production Example 4) | 125 | 251 | 126 |
| Example 5 | Esterification reaction product (Production Example 5) | 123 | 345 | 222 |
| Example 6 | Esterification reaction product (Production Example 6) | 121 | 351 | 230 |
| Example 7 | Esterification reaction product (Production Example 7) | 130 | 323 | 193 |
| Example 8 | Esterification reaction product (Production Example 8) | 131 | 452 | 321 |
| Example 9 | Esterification reaction product (Production Example 9) | 123 | 288 | 165 |

[Table 3-2]

| Sample | | Before application | After application | Resistance value |
|--------|--|--------------------|------------------|------------------|
| Comparative Example 1 | Esterification reaction product (Production Comparative Example 1) | 130 | 326 | 196 |
| Comparative Example 2 | Esterification reaction product (Production Comparative Example 2) | 130 | 265 | 135 |
| Comparative Example 3 | Octyl oxystearate | 149 | 201 | 52 |
| Comparative Example 4 | Dipentaerythrite fatty acid ester (12-hydroxystearic acrid : isostearic acid : hydrogen =3:1:2) | 108 | 620 | 512 |
| Comparative Example 5 | Dipentaerythrityl tri-polyhydroxystearate | 115 | 243 | 132 |
| Comparative Example 6 | Trimethylsiloxysilicate (water-repellent silicone) | 123 | 170 | 56 |
| Comparative Example 7 | Diisostearyl malate | 118 | 364 | 246 |
| Comparative Example 8 | Diglyceryl triisostearate | 118 | 274 | 156 |

In Table 3, a higher resistance value means a larger amount of moisture. In this test, a resistance value of about 240 $\mu$S is the optimum value. A resistance value of 51.2 $\mu$S is too high and may affect other factors.

As obvious from the results of the conductance measurement test shown in Table 3, the resistance values of the esterification reaction products prepared in Production Examples 1 to 3 and 5 to 7 were each about 240 $\mu$S and were thus optimum values.

Next, cosmetic products were produced and were evaluated by a sensory test shown below.

Sensory evaluation of cosmetic product

Ten sensory evaluation panelists used each cosmetic product and evaluated for sheen (whether natural sheen is provided to the portion such as the lips to which the cosmetic product is applied), easiness of application (whether the easiness of application when the cosmetic product is used is satisfactory), moist feeling (whether moisture is provided to the portion to which the cosmetic product is applied), makeup-lasting quality (whether the makeup-lasting quality when the cosmetic product is used is satisfactory), and stickiness (whether the cosmetic product is sticky when it is used). The evaluation factors were graded by the following criteria:

Good: 2,
Moderate: 1, and
Poor: 0.

The cosmetic products were each evaluated by the sum of the points of ten panelists as follows:

A: 15 to 20,
B: 10 to 14
C: 5 to 9, and
D: 4 or less.

Here, the excellent functions of the cosmetic products containing the esterification reaction product of the present invention, i.e., waterproof- film- forming properties, adhesion to, for example, skin, and moisturizing properties will be described.

In the sensory evaluation of the cosmetic products, excellent waterproof- film- forming properties lead to good moist feeling and an improvement in easiness of application; high adhesion to, for example, skin leads to an improvement in makeup- lasting quality; and excellent moisturizing properties lead to an improvement in moist feeling. Examples 10 to 18 and Comparative Examples 9 to 14 Production of lip gloss

Lip glosses having compositions shown in Tables 4 to 6 were produced by the method shown below. The unit of the numbers in the tables is % by mass. Table 4 also shows the evaluation results.

The compositional raw materials were weighed and were melted in a water bath at 90°C and were then stirred for 2 minutes, deaerated, and loaded to provide the respective lip glosses.

[Table 4-1]

| Compositional raw material | Example 10 | Example 11 | Example 12 | Example 13 |
|---|---|---|---|---|
| 1. Diisostearyl malate | 20 | 20 | 20 | 20 |
| 2. Hydrogenated polyisobutene | 50 | 50 | 50 | 50 |
| 3. Pentaerythrite tetraisostearate | 18 | 18 | 18 | 18 |
| 4. Esterification reaction product (Production Example 1) | 10 | - | - | - |
| 5. Esterification reaction product (Production Example 2) | - | 10 | - | - |
| 6. Esterification reaction product (Production Example 3) | - | - | 10 | - |
| 7. Esterification reaction product (Production Example 4) | - | - | - | 10 |

(continued)

| Compositional raw material | Example 10 | Example 11 | Example 12 | Example 13 |
|---|---|---|---|---|
| 8. Glycerin fatty acid ester eicosadioate condensate | 2 | 2 | 2 | 2 |
| Total | 100 | 100 | 100 | 100 |
| Evaluation | | | | |
| Sheen | A | A | B | B |
| Easiness of application | A | B | B | C |
| Moisture | A | A | A | B |
| Makeup-lasting quality | A | A | A | A |
| Stickiness | A | B | B | B |

[Table 4-2]

| Compositional raw material | Example 14 | Example 15 | Example 16 | Example 17 | Example 18 |
|---|---|---|---|---|---|
| 1. Diisostearyl malate | 20 | 20 | 20 | 20 | 20 |
| 2. Hydrogenated polyisobutene | 50 | 50 | 50 | 50 | 50 |
| 3. Pentaerythrite tetraisostearate | 18 | 18 | 18 | 18 | 18 |
| 4. Esterification reaction product (Production Example 5) | 10 | - | - | - | - |
| 5. Esterification reaction product (Production Example 6) | - | 10 | - | - | - |
| 6. Esterification reaction product (Production Example 7) | - | - | 10 | - | - |
| 7. Esterification reaction product (Production Example 8) | - | - | - | 10 | - |
| 8. Esterification reaction product (Production Example 9) | | | | | 10 |
| 9. Glycerin fatty acid ester eicosadioate condensate | 2 | 2 | 2 | 2 | 2 |
| Total | 100 | 100 | 100 | 100 | 100 |
| Evaluation | | | | | |
| Sheen | A | A | B | A | A |
| Easiness of application | A | B | A | B | A |
| Moisture | A | A | B | A | A |
| Makeup-lasting quality | A | A | A | A | B |
| Stickiness | A | A | A | B | A |

In the table, the diisostearyl malate used was Cosmol 222 (trade name) manufactured by The Nisshin OilliO Group, Ltd., the pentaerythrityl tetraisostearate used was Salacos 5418V (trade name) manufactured by The Nisshin OilliO Group, Ltd., the octyl oxystearate used was Salacos EH (trade name) manufactured by The Nisshin OilliO Group, Ltd., the dipentaerythrite fatty acid ester (12-hydroxystearic acid : isostearic acid : hydrogen = 3:1:2) used was Cosmol 168EV (trade name) manufactured by The Nisshin OilliO Group, Ltd.), the dipentaerythrityl tri-polyhydroxystearate used was Salacos WO-6 (trade name) manufactured by The Nisshin OilliO Group, Ltd., the trimethylsiloxysilicate used was X-21-5250 (trade name) manufactured by Shin-Etsu Chemical Co., Ltd., and glycerin fatty acid ester eicosadioate condensate used was Nomcort HK-G (trade name) manufactured by The Nisshin OilliO Group, Ltd. These are the same in the following tables.

[Table 5]

| Compositional raw material | Comparative Example 9 | Comparative Example 10 |
|---|---|---|
| 1. Diisostearyl malate | 20 | 20 |
| 2. Hydrogenated polyisobutene | 50 | 50 |
| 3. Pentaerythrite tetraisostearate | 18 | 18 |
| 4. Esterification reaction product (Production Comparative Example 1) | 10 | - |
| 5. Esterification reaction product (Production Comparative Example 2) | - | 10 |
| 6. Glycerin fatty acid ester eicosadioate condensate | 2 | 2 |
| Total | 100 | 100 |
| Evaluation | | |
| Sheen | B | B |
| Easiness of application | C | B |
| Moisture | B | C |
| Makeup-lasting quality | B | C |
| Stickiness | C | B |

[Table 6]

| Compositional raw material | Comparative Example 11 | Comparative Example 12 | Comparative Example 13 | Comparative Example 14 |
|---|---|---|---|---|
| 1. Diisostearyl malate | 20 | 20 | 20 | 20 |
| 2. Hydrogenated polyisobutene | 50 | 50 | 50 | 50 |
| 3. Pentaerythrite tetraisostearate | 18 | 18 | 18 | 18 |
| 4. Octyl oxystearate | 10 | - | - | - |
| 5. Dipentaerythrite fatty acid ester (12-hydroxystearic acid : isostearic acid : hydrogen = 3:1:2) | - | 10 | - | - |
| 6. Dipentaerythrityl tri-polyhydroxystearate | - | - | 10 | - |
| 7. Trimethylsiloxysilicate (water-repellent silicone) | - | - | - | 10 |
| 8. Glycerin fatty acid ester eicosadioate condensate | 2 | 2 | 2 | 2 |
| Total | 100 | 100 | 100 | 100 |

(continued)

| Evaluation | | | | |
|---|---|---|---|---|
| Sheen | D | D | B | D |
| Easiness of application | B | D | D | A |
| Moisture | B | C | B | C |
| Makeup-lasting quality | D | B | A | D |
| Stickiness | A | D | D | A |

As obvious from Tables 4 to 6, the lip glosses produced using the esterification reaction product of the present invention showed excellent moisture, makeup-lasting quality, and stickiness in the tests. In contrast, the lip glosses of Comparative Examples were inferior to the lip glosses in Examples in all factors devaluated.

Example 19

Production of lipstick

A lipstick having the composition shown in Table 7 was produced by the following method.
Components 1 to 11 were uniformly melted and mixed, and components 12 to 15 were added thereto, followed by kneading. Subsequently, the mixture was remelted, and components 16 to 18 were added thereto, followed by uniformly dispersing and mixing. The mixture was deaerated and was poured into a mold. After cooling and solidification, the product was accommodated in a container to give a lipstick.
[Table 7]

| Compositional raw material | Example 19 |
|---|---|
| 1. Paraffin | 6.0 |
| 2. Synthetic hydrocarbon wax | 2.0 |
| 3. Microcrystalline wax | 3.0 |
| 4. Hydrogenated polyisobutene | 30.0 |
| 5. Esterification reaction product (Production Example 1) | 10.0 |
| 6. Di(phytosteryl/octyldodecyl) lauroyl glutamate | 10.0 |
| 7. Diglyceryl triisostearate | 5.0 |
| 8. Octyl dodecanol | 5.0 |
| 9. $\alpha$-Olefin oligomer | 10.0 |
| 10. Glyceryl tri(capryl/caprate) | residual quantity |
| 11. Tocopherol | 0.1 |
| 12. Red No. 202 | 0.3 |
| 13. Yellow No. 4 aluminum lake | 0.2 |
| 14. Bengara | 0.4 |
| 15. Titanium oxide | 1.0 |
| 16. Mica titanium | 3.0 |
| 17. Hydrophobized mica titanium | 2.5 |
| 18. Titanium oxide-coated glass flake | 2.0 |
| Total | 100 |

Example 20

Production of lip gloss

A lip gloss having the composition shown in Table 8 was produced by the following method.

Components 1 to 8 were uniformly melted and mixed, and components 9 to 11 were added thereto, followed by kneading. Subsequently, the mixture was remelted, and components 12 to 14 were added thereto, followed by uniformly dispersing and mixing. The mixture was deaerated and was loaded in a container to give a lip gloss.

[Table 8]

| Compositional raw material | Example 20 |
|---|---|
| 1. Dextrin palmitate | 5.0 |
| 2. Hydrogenated polyisobutene | 40.0 |
| 3. Esterification reaction product (Production Example 1) | 10.0 |
| 4. Diisostearyl malate | 10.0 |
| 5. Di(phytosteryl/octyldodecyl) lauroyl glutamate | 5.0 |
| 6. Squalane | 5.0 |
| 7. Octyl hydroxystearate | residual quantity |
| 8. Tocopherol | 0.1 |
| 9. Anhydrous silicic acid (Aerosil 200V, manufactured by Nippon Aerosil Co., Ltd.) | 1.0 |
| 10. Red No. 202 | 0.1 |
| 11. Yellow No. 4 aluminum lake | 0.1 |
| 12. Mica titanium | 1.0 |
| 13. Titanium oxide-coated glass flake | 1.0 |
| 14. (PET/methyl polymethacrylate) laminate | 0.5 |
| Total | 100 |

Example 21

Production of solid powder eye shadow

A solid powder eye shadow having the composition shown in Table 9 was produced by the following method. Components 1 to 3 were mixed and melted. Separately, components 4 to 15 were uniformly mixed, and the melted mixture of components 1 to 3 was added thereto, followed by uniformly dispersing and pulverizing to give a cosmetic product base material. A volatile solvent (isododecane) was mixed with the resulting cosmetic product base material in an amount 50 parts by mass based on 100 parts by mass of the base material. The mixture was placed in a metal dish, followed by removing the solvent by drying with heat to give a solid powder eye shadow.

[Table 9]

| Compositional raw material | Example 21 |
|---|---|
| 1. Methylphenylpolysiloxane | 2.0 |
| 2. Esterification reaction product (Production Example 1) | 5.0 |
| 3. Glyceryl tri(capryl/caprate) | 4.0 |
| 4. Mica | residual quantity |
| 5. Aluminum stearate | 2.0 |

(continued)

| Compositional raw material | Example 21 |
|---|---|
| 6. Red No. 202 | 0.5 |
| 7. Yellow No. 4 aluminum lake | 3.0 |
| 8. Mica titanium | 30.0 |
| 9. Silica-coated aluminum powder | 10.0 |
| 10. Titanium oxide-coated glass powder | 20.0 |
| 11. Silver-coated glass powder | 5.0 |
| 12. Silicone (5%) treated mica titanium | 10.0 |
| 13. Tocopherol | 0.1 |
| 14. Methylparaben | 0.2 |
| 15. Sodium dehydroacetate | 0.1 |
| Total | 100 |

Example 22

Production of solid powder foundation

A foundation having the composition shown in Table 10 was produced by the following method.
Components 1 to 4 were mixed and melted. Separately, components 5 to 15 were uniformly mixed, and the melted mixture of components 1 to 4 was added thereto, followed by uniformly dispersing and pulverizing to give a cosmetic product base material. The resulting cosmetic product base material was placed in a metal dish and pressed to give a solid powder foundation. In the table, the cross-linked silicone powder used was KSP-300 manufactured by Shin-Etsu Chemical Co., Ltd.
[Table 10]

| Compositional raw material | Example 22 |
|---|---|
| 1. Dimethylpolysiloxane | 2.0 |
| 2. Esterification reaction product (Production Example 1) | 3.0 |
| 3. Isononyl isononanoate | 3.0 |
| 4. 2-Ethylhexyl paramethoxycinnamate | 2.0 |
| 5. Silicone-treated bengara | 0.5 |
| 6. Silicone-treated yellow iron oxide | 2.0 |
| 7. Silicone-treated black iron oxide | 0.3 |
| 8. Fluorine-treated titanium oxide | 15.0 |
| 9. Lauroyl lysine-treated mica | 25.0 |
| 10. Zinc myristate-treated talc | residual quantity |
| 11. Hemp cellulose powder | 5.0 |
| 12. Cross-linked silicone powder | 3.0 |
| 13. Chlorphenesin | 0.1 |
| 14. Methylparaben | 0.2 |
| 15. Sodium dehydroacetate | 0.1 |
| Total | 100 |

Example 23

Production of cream

A cream having the composition shown in Table 11 was produced by the following method. Components 1 to 14 were melted at 80°C. Separately, components 15 to 28 were melted at 80°C. Two component mixtures were mixed with each other and cooled to 30°C with stirring, and the resulting mixture was loaded in a container to give a cream.

[Table 11]

| Compositional raw material | Example 23 |
| --- | --- |
| 1. Esterification reaction product (Production Example 1) | 3.0 |
| 2. Stoaric acid | 1.0 |
| 3. Isostearic acid | 0.2 |
| 4. Glyceryl monostearate | 2.0 |
| 5. Behenyl alcohol | 2.0 |
| 6. Bees wax | 1.0 |
| 7. Cholesterol | 0.2 |
| 8. Macadamia nut oil fatty acid phytosteryl | 1.0 |
| 9. Sorbitan stearate | 0.5 |
| 10. Ceramide 2 | 0.03 |
| 11. Hydrogenated lecithin | 0.1 |
| 12. Plant squalane | 8.0 |
| 13. Macadamia nut oil | 5.0 |
| 14. Magnolignan | 0.01 |
| 15. 1,3-Butylene glycol | 5.0 |
| 16. Concentrated glycerin | 5.0 |
| 17. Methylparaben | 0.2 |
| 18. Dipotassium glycyrrhizinate | 0.1 |
| 19. Pyrus communis fruit juice fermentation extract | 0.2 |
| 20. N-acetylglucosamine | 0.1 |
| 21. Bilberry leaf extract | 0.1 |
| 22. Glucoside ascorbate | 0.2 |
| 23. γ-Amino butyric acid | 0.1 |
| 24. Sodium N-stearoyl glutamate | 0.2 |
| 25. Alkyl-modified carboxyvinyl polymer | 0.05 |
| 26. Nicotinamide | 0.1 |
| 27. Chlorphenesin | 0.1 |
| 28. Purified water | residual quantity |
| Total | 100 |

Example 24

Production of W/O cream

A W/O cream having the composition shown in Table 12 was produced by the following method. Components 10 to 25 were uniformly melted at 60°C. Separately, components 1 to 9 were uniformly melted at 60°C and were added to the melted mixture of components 10 to 25, followed by emulsification and dispersion. Then, the resulting emulsion was cooled to 30°C to give a W/O cream. In the table, co-modified silicon used was ABIL EM90 manufactured by Goldschmidt A.G., the POE-modified silicon dispersion used was Silicon BY22-008 manufactured by Toray Dow Corning Silicone Co., Ltd., the long-chain branched fatty acid cholesteryl used was YOFCO CLE-NH manufactured by Nippon Fine Chemical Co., and the silicon elastomer dispersion used was Trefil E-508 manufactured by Toray Dow Corning Silicone Co., Ltd.

[Table 12]

| Compositional raw material | Example 24 |
|---|---|
| 1. Esterification reaction product (Production Example 1) | 2.0 |
| 2. Co-modified silicon | 2.0 |
| 3. POE-modified silicon dispersion | 1.0 |
| 4. Squalane | 5.0 |
| 5. Octyl dodecyl myristate | 5.0 |
| 6. Methylpolysiloxane (100 cs) | 3.0 |
| 7. Long-chain branched fatty acid cholesteryl | 1.0 |
| 8. Silicon elastomer dispersion | 5.0 |
| 9. Coenzyme Q10 | 0.03 |
| 10. Pycnogenol | 0.03 |
| 11. Camu camu extract | 0.1 |
| 12. Rice fermentation extract | 1.0 |
| 13. Orchid extract | 0.1 |
| 14. Okra extract | 0.1 |
| 15. Oolong tea extract | 0.1 |
| 16. Jojoba leaf extract | 0.1 |
| 17. Licorice extract | 0.1 |
| 18. Water-soluble chlorophyll | 0.02 |
| 19. Sodium chloride | 1.0 |
| 20. Dipropylene glycol | 5.0 |
| 21. Concentrated glycerin | 5.0 |
| 22. Raffinose | 1.0 |
| 23. Methylparaben | 0.3 |
| 24. N-Methyl-L-serine | 0.5 |
| 25. Purified water | residual quantity |
| Total | 100 |

Example 25

Production of gel

A gel having the composition shown in Table 13 was produced by the following method.
Components 1 to 10 were melted at 60°C. Separately, components 11 to 25 were melted at 60°C. Two component mixtures were mixed with each other and cooled to 30°C with stirring to give a gel. In the table, the spherical silicon powder used was Tospearl 145A manufactured by Momentive Performance Materials Inc. and the ceramide saccharide used was Bioceramide manufactured by Kibun Food Chemical Co.

[Table 13]

| Compositional raw material | Example 25 |
|---|---|
| 1. Esterification reaction product (Production Example 1) | 1.0 |
| 2. Dimethylpolysiloxane (6 cs) | 10.0 |
| 3. Olive oil | 0.1 |
| 4. Macadamia nut oil | 0.1 |
| 5. Jojoba oil | 0.1 |
| 6. Retinyl palmitate | 0.1 |
| 7. di-α-Tocopherol nicotinate | 0.1 |
| 8. POE-hydrogenated castor oil (60 E.O.) | 2.0 |
| 9. Spherical silicon powder | 1.0 |
| 10. Ascorbyl tetraisopalmitate | 0.5 |
| 11. Hydrolyzed hyaluronic acid | 0.1 |
| 12. Stevia extract | 0.2 |
| 13. Ethanol | 5.0 |
| 14. Rhododendrol | 0.1 |
| 15. Phellodendron bark extract | 0.1 |
| 16. 1,3-Butylene glycol | 5.0 |
| 17. Sorbitol liquid | 3.0 |
| 18. Polyethylene glycol 4000 | 1.0 |
| 19. Carboxyvinyl polymer | 0.2 |
| 20. Ceramide saccharide | 0.1 |
| 21. Methylparaben | 0.2 |
| 22. γ-Amino-β-hydroxybutyric acid | 0.2 |
| 23. Disodium edetate | 0.02 |
| 24. Potassium hydroxide | 0.02 |
| 25. Purified water | residual quantity |
| Total | 100 |

Example 26

Production of milky lotion

A milky lotion having the composition shown in Table 14 was produced by the following method.

Components 1 to 7 were melted at 60C. Separately, components 8 to 26 were melted at 60°C. Two component mixtures were mixed with each other and cooled to 30°C with stirring to give a milky lotion.

[Table 14]

| Compositional raw material | Example 26 |
|---|---|
| 1. Esterification reaction product (Production Example 1) | 0.5 |
| 2. Methylphenylpolysiloxane | 3.0 |
| 3. POE-hydrogenated castor oil (60 E.O.) | 0.8 |
| 4. Polyoxyethylene coconut oil fatty acid sorbitan | 0.2 |
| 5. Phenoxyethanol | 0.2 |
| 6. Dipropylene glycol | 3.0 |
| 7. Ethanol | 6.0 |
| 8. Tremella fuciformis polysaccharide | 0.1 |
| 9. Xanthan gum | 0.3 |
| 10. Sodium citrate | 0.05 |
| 11. Equisetum arvense extract | 0.1 |
| 12. 1,3-Butylene glycol | 5.0 |
| 13. Carrageenan | 0.1 |
| 14. Pycnogenol | 0.05 |
| 15. Bitter orange peel extract | 0.1 |
| 16. Carboxyvinyl polymer | 0.2 |
| 17. Watercress extract | 0.1 |
| 18. Avocado extract | 0.2 |
| 19. Oenothera biennis extract | 0.2 |
| 20. Mevalonolactone | 0.1 |
| 21. Sodium hyaluronate | 0.001 |
| 22. Sodium glycyrrhizinate | 0.2 |
| 23. Potassium hydroxide | 0.05 |
| 24. Chlorphenesin | 0.05 |
| 25. Perfume | 0.02 |
| 26. Purified water | residual quantity |
| Total | 100 |

Example 27

Production of liquid foundation

A liquid foundation having the composition shown in Table 15 was produced by the following method. Components 1 to 8 were mixed and uniformly melted at 80°C, and pulverized components 9 to 11 were added thereto. Components 12 and 13 were mixed and heated to 80°C and were added to the mixture prepared above. The resulting mixture was cooled to room temperature with mixing to give a liquid foundation.

[Table 15]

| Compositional raw material | Example 27 |
|---|---|
| 1. Sorbitan isostearate | 1 |
| 2. Dimethicone copolyol | 1 |
| 3. Cyclomothicone | 15 |
| 4. Dimethicone | 5 |
| 5. Mineral oil | 5 |
| 6. Esterification reaction product (Production Example 1) | 2 |
| 7. Octyl methoxycinnamate | 3 |
| 8. Microcrystalline wax | 1 |
| 9. Talc | 5 |
| 10. Titanium oxide | 8 |
| 11. Iron oxide | 2 |
| 12. Glycerin | 4 |
| 13. Purified water | residual quantity |
| Total | 100 |

Examples 28 to 30

Production of hair conditioner

Hair conditioners having the compositions shown in Table 16 were produced by the following method. Components 1 to 8 were mixed and uniformly melted at 80°C. Separately, components 10 to 13 were heated to 55 to 80°C and were mixed. The mixture was added to the melted mixture of components 1 to 8. The resulting mixture was cooled, and component 9 was added thereto to give each hair conditioner.
[Table 16]

| Compositional raw material | Example 28 | Example 29 | Example 30 |
|---|---|---|---|
| 1. Behen trimonium chloride | 1.5 | | |
| 2. Octadecyloxy(2-hydroxypropyl)dimethylamine | | 1.5 | |
| 3. Dimethylaminopropylamide stearate | | | 1.5 |
| 4. Stearyl alcohol | 4.5 | 6 | 6 |
| 5. Behenyl alcohol | 1.5 | | |
| 6. Benzyl alcohol | | | 0.3 |
| 7. Dipropylene glycol | | | 1.5 |
| 8. Esterification reaction product (Production Example 1) | 0.5 | 0.5 | 0.5 |
| 9. Dimethicone | 2 | 2 | 2 |
| 10. Lactic acid | | 1 | 1.5 |
| 11. Glycolic acid | | 0.2 | |
| 12. Glutamic acid | | 0.3 | |
| 13. Deionized water | residual quantity | residual quantity | residual quantity |
| Total | 100 | 100 | 100 |

Example 31

Production of concealer

A concealer having the composition shown in Table 17 was produced by the following method. Components 1 to 11 were melted with heat and uniformly mixed, and components 12 to 15 were added thereto. The mixture was kneaded with a roll mill for uniformly dispersing. The resulting dispersion was remelted together with components 16 and 17. After deaeration, the mixture was poured into a mold and was cooled to room temperature to give a concealer.

[Table 17]

| Compositional raw material | Example 31 |
|---|---|
| 1. Paraffin wax | 5 |
| 2. Polyethylene wax | 3 |
| 3. Microcrystalline wax | 5 |
| 4. Carnauba wax | 3 |
| 5. Candelilla wax | 3 |
| 6. Esterification reaction product (Production Example 1) | 0.5 |
| 7. Diisostearyl malate | 15 |
| 8. Liquid paraffin | 10 |
| 9. Olive oil | 5 |
| 10. Neopentyl glycol dicaprate | residual quantity |
| 11. Dibutyl hydroxytoluene | 0.03 |
| 12. Titanium oxide | 20 |
| 13. Bengara | 0.5 |
| 14. Yellow iron oxide | 2.5 |
| 15. Black iron oxide | 0.2 |
| 16. Mica | 3 |
| 17. Mica titanium | 5 |
| Total | 100 |

Example 32

Production of lipstick

A lipstick having the composition shown in Table 18 was produced by the following method. Components 1 to 13 were uniformly melted and mixed, and components 14 to 17 were added thereto, followed by kneading. Subsequently, the mixture was remelted, and components 18 to 20 were added thereto, followed by uniformly dispersing and mixing. The mixture was deaerated and was poured into a mold. After cooling and solidification, the product was accommodated in a container to give a lipstick.

[Table 18]

| Compositional raw material | Example 32 |
|---|---|
| 1. Paraffin | 5.0 |
| 2. Ethylene propylene copolymer | 3.0 |

(continued)

| Compositional raw material | Example 32 |
|---|---|
| 3. Microcrystalline wax | 2.0 |
| 4. Hydrogenated polyisobutene | 10.0 |
| 5. Esterification reaction product (Production Example 1) | 30.0 |
| 6. Di(phytosteryl/octyldodecyl) lauroyl glutamate | 10.0 |
| 7. Diglyceryl triisostearate | 5.0 |
| 8. Isotridecyl isononanoate | 5.0 |
| 9. α-Olefin oligomer | 10.0 |
| 10. Glyceryl tri(capryl/caprate) | residual quantity |
| 11. Diglyceryl diisostearate | 0.5 |
| 12. Apricot extract | 0.1 |
| 13. Tocopherol | 0.1 |
| 14. Red No. 202 | 0.3 |
| 15. Yellow No. 4 aluminum lake | 0.2 |
| 16. Bengara | 0.4 |
| 17. Titanium oxide | 1.0 |
| 18. Mica titanium | 3.0 |
| 19. Hydrophobized mica titanium | 2.5 |
| 20. Titanium oxide-coated glass flake | 2.0 |
| Total | 100 |

Example 33

Production of liquid rouge

A liquid rouge having the composition shown in Table 19 was produced by the following method. Components 1 to 9 were uniformly melted and mixed, and components 10 to 15 were added thereto, followed by kneading. Subsequently, the mixture was remelted, and components 16 to 17 were added thereto, followed by uniformly dispersing and mixing. The mixture was deaerated and was loaded in a container to give a liquid rouge.

[Table 19]

| Compositional raw material | Example 33 |
|---|---|
| 1. Dextrin palmitate | 1.0 |
| 2. Glycerin fatty acid ester eicosadioate condensate | 3.0 |
| 3. Hydrogenated polyisobutene | 5.0 |
| 4. Esterification reaction product (Production Example 1) | 35.0 |
| 5. Dipentaerythrityl pentaisostearate | 5.0 |
| 6. Di(phytosteryl/octyldodecyl) lauroyl glutamate | 10.0 |
| 7. Diglyceryl triisostearate | 15.0 |
| 8. Octyl hydroxystearate | residual quantity |
| 9. Tocopherol | 0.1 |

(continued)

| Compositional raw material | Example 33 |
|---|---|
| 10. Red No. 202 | 0.1 |
| 11. Bengara | 0.1 |
| 12. Yellow iron oxide | 0.2 |
| 13. Black iron oxide | 0.1 |
| 14. Titanium oxide | 1.0 |
| 15. Plate-like barium sulfate | 3.0 |
| 16. Mica titanium | 4.0 |
| 27. Titanium oxide-coated glass flake | 1.0 |
| Total | 100 |

The liquid rouge of Example 33 was evaluated by five panelists for sheen (whether natural sheen is provided to the portion to which the liquid rouge applied), easiness of application (whether the easiness of application of the liquid rouge is satisfactory), moist feeling (whether moisture is provided to the portion to which the liquid rouge is applied), makeup-lasting quality (whether the makeup-lasting quality of the liquid rouge is satisfactory), and stickiness (whether the liquid rouge is sticky when it is used). The sensory evaluation was performed by the following criteria, and the results are shown in Table 20.
Evaluation criteria:

A: four or more panelists recognized the effect,
B: three or more panelists recognized the effect,
C: two or more panelists recognized the effect, and
D: one or less panelist recognized the effect.

[Table 20]

| Evaluation factor | Evaluation result |
|---|---|
| Sheen (whether natural sheen is provided to the portion to which the liquid rouge applied) | A |
| Easiness of application (whether the easiness of application of the liquid rouge is satisfactory) | A |
| Moist feeling (whether moisture is provided to the portion to which the liquid rouge is applied) | A |
| Makeup-lasting quality (whether the makeup-lasting quality of the liquid rouge is satisfactory) | A |
| Stickiness (whether the liquid rouge is sticky when it is used) | A |

In addition, the cosmetic products produced in Examples 14 to 32 also gave satisfactory evaluation results as cosmetic products.

**Claims**

1. An esterification reaction product prepared by esterifying the following component A, component B, and component C:

component A: 12-hydroxystearic acid polymer having a degree of polymerization of 7 or more,
component B: fatty acid other than 12-hydroxystearic acid polymers, and
component C: pentaerythritol.

2. The esterification reaction product according to Claim 1, having a hydroxy value of 10 to 70 mgKOH/g and an acid value of 10 mgKOH/g or less.

3. The esterification reaction product according to Claim 1 or 2, having an acid value of 3 mgKOH/g or less.

4. The esterification reaction product according to any one of Claims 1 to 3, having a hydroxy value of 10 to 30 mgKOH/g.

5. The esterification reaction product according to any one of Claims 1 to 4, having fatty acid residues in which the molar ratio (%) of component A is 35 to 90%, and the molar ratio (%) of component B is 10 to 65%.

6. The esterification reaction product according to any one of Claims 1 to 5, wherein the component B is a saturated fatty acid.

7. The esterification reaction product according to any one of Claims 1 to 5, wherein component B is a linear saturated fatty acid having 16 to 28 carbon atoms.

8. The esterification reaction product according to any one of Claims 1 to 5, wherein component B is behenic acid.

9. The esterification reaction product according to any one of Claims 1 to 8, wherein component A has a degree of polymerization of 8 to 20.

10. The esterification reaction product according to any one of Claims 1 to 9, wherein component A is prepared by polymerizing 12-hydroxystearic acid having a purity of 94% by mass or more.

11. A cosmetic product comprising an esterification reaction product according to any one of Claims 1 to 10.

12. The cosmetic product according to Claim 11, comprising the esterification reaction product in an amount of 0.1 to 80% by mass.

13. The cosmetic product according to Claim 11 or 12, being selected from the group consisting of lip cosmetic products, foundations, emollient creams, milky lotions, bases, hair creams, shampoos, hair rinses, hair conditioners, hand creams, beauty essences, eye-area cosmetic products, nail cosmetic products, and sunscreen cosmetic products.

14. A method of producing an esterification reaction product, the method comprising:

    esterifying the following component A, component B, and component C:
    component A: 12-hydroxystearic acid polymer having a degree of polymerization of 7 or more,
    component B: fatty acid other than 12-hydroxystearic acid polymers, and
    component C: pentaerythritol.

15. The method of producing an esterification reaction product according to Claim 14, the method comprising:

    preparing component A by polymerizing 12-hydroxystearic acid; and
    esterifying the resulting component A, component B, and component C.

16. The method of producing an esterification reaction product according to Claim 14 or 15, wherein component B is behenic acid; and component A is prepared by polymerizing 12-hydroxystearic acid having a purity of 94% by mass or more.

EP 2 639 253 A1

## INTERNATIONAL SEARCH REPORT

International application No.

PCT/JP2011/075400

### A. CLASSIFICATION OF SUBJECT MATTER

*C08G63/06*(2006.01)i, *A61K8/85*(2006.01)i, *A61Q1/00*(2006.01)i, *A61Q5/00*(2006.01)i, *A61Q19/00*(2006.01)i, *C07C69/675*(2006.01)i

According to International Patent Classification (IPC) or to both national classification and IPC

### B. FIELDS SEARCHED

Minimum documentation searched (classification system followed by classification symbols)
C08G63/06, A61K8/85, A61Q1/00, A61Q5/00, A61Q19/00, C07C69/675

Documentation searched other than minimum documentation to the extent that such documents are included in the fields searched

| | | |
|---|---|---|
| Jitsuyo Shinan Koho | 1922-1996 | Jitsuyo Shinan Toroku Koho 1996-2012 |
| Kokai Jitsuyo Shinan Koho | 1971-2012 | Toroku Jitsuyo Shinan Koho 1994-2012 |

Electronic data base consulted during the international search (name of data base and, where practicable, search terms used)
CA(STN)

### C. DOCUMENTS CONSIDERED TO BE RELEVANT

| Category* | Citation of document, with indication, where appropriate, of the relevant passages | Relevant to claim No. |
|---|---|---|
| A | WO 2006/080389 A1 (The Nisshin Oillio Group, Ltd.), 03 August 2006 (03.08.2006), entire text & US 2008/0260663 A1 & EP 1857433 A1 & CN 101107214 A & KR 10-2007-0104374 A | 1-16 |
| A | WO 2007/059888 A2 (COGNIS IP MANAGEMENT GMBH), 31 May 2007 (31.05.2007), entire text & US 2009/0082284 A1 & EP 1813311 A1 & EP 1954354 A | 1-16 |
| A | JP 2001-247842 A (The Nisshin Oil Mills, Ltd.), 14 September 2001 (14.09.2001), entire text (Family: none) | 1-16 |

☐ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 January, 2012 (30.01.12) | 07 February, 2012 (07.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP SHO54109917 B
- JP SHO5557509 B
- JP SHO56115740 B
- JP SHO56108739 B

- JP HEI05331023 B
- JP SHO6490025 B
- JP 2000290232 A
- JP HEI078781 B

**Non-patent literature cited in the description**

- Keshohin Jiten. Maruzen, 15 December 2003